(19) Europäisches Patentamt
European Patent Office
Office européen des brevets

(11) EP 3 486 247 A1

(12) **EUROPEAN PATENT APPLICATION**
published in accordance with Art. 153(4) EPC

(43) Date of publication:
**22.05.2019 Bulletin 2019/21**

(21) Application number: **17827635.8**

(22) Date of filing: **11.07.2017**

(51) Int Cl.:
*C07D 495/16* (2006.01) *C07D 519/00* (2006.01)
*C07F 7/10* (2006.01) *H01L 29/786* (2006.01)
*H01L 51/05* (2006.01) *H01L 51/30* (2006.01)
*H01L 51/46* (2006.01)

(86) International application number:
**PCT/JP2017/025308**

(87) International publication number:
**WO 2018/012508 (18.01.2018 Gazette 2018/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **12.07.2016 JP 2016137822**

(71) Applicants:
- **Riken**
**Wako-shi, Saitama 351-0198 (JP)**
- **Nippon Kayaku Kabushiki Kaisha**
**Tokyo 100-0005 (JP)**

(72) Inventors:
- **NAKANO Masahiro**
**Wako-shi**
**Saitama 351-0198 (JP)**
- **KIM Ji-Hoon**
**Wako-shi**
**Saitama 351-0198 (JP)**
- **TAKIMIYA Kazuo**
**Wako-shi**
**Saitama 351-0198 (JP)**
- **NAKAMURA Hiroshi**
**Tokyo 115-8588 (JP)**

(74) Representative: **Gille Hrabal**
**Brucknerstrasse 20**
**40593 Düsseldorf (DE)**

# (54) AROMATIC COMPOUND AND USE THEREOF

(57) There are provided a novel aromatic compound which has excellent semiconductor properties in terms of carrier mobility, an organic semiconductor material and a thin film forming composition containing the compound, an organic thin film containing the compound, and an organic semiconductor device containing the thin film. The aromatic compound is represented by General Formula (1).

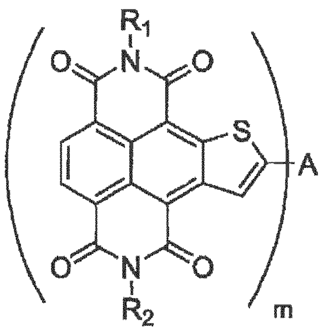

(1)

In General Formula (1), $R_1$ and $R_2$ independently represent a hydrogen atom, or a saturated hydrocarbon group having 1 to 30 carbon atoms, a saturated fluorohydrocarbon group having 1 to 30 carbon atoms, an aryl group, or a styryl group that may have a substituent; m is an integer from 1 to 6; and A represents a hydrogen atom, a halogen atom, a saturated hydrocarbon-trisubstituted silyl group, an aryl group, a heteroaryl group, a direct bond, or a di- to hexavalent linking group having an aromatic ring or a heterocyclic ring.

EP 3 486 247 A1

**Description**

Technical Field

The present invention relates to novel aromatic compounds and use thereof. More specifically, the present invention relates to a novel naphtho[2,3-b]thiophene diimide derivative which can be used as an organic semiconductor or the like, and an organic semiconductor material, a thin film forming composition, an organic thin film, and an organic semiconductor device using the novel derivative.

Background Art

Naphthalene diimide (hereinafter may be abbreviated as "NDI") has been widely employed as an electron-deficient organic semiconductor skeleton (as an acceptor, or an n-type semiconductor). NDI with extended conjugation can be utilized as various materials. According to some reports, an NDI skeleton is incorporated via a single bond into a conjugated system of an oligomer or a polymer, and a resulting compound is used to fabricate n-type semiconductors, p-type semiconductors, or ambipolar semiconductors.

Recent researches also consider n-extended NDI derivatives in which an aromatic ring is directly condensed with an NDI skeleton. PTL 1 and NPL 1 to 5 synthesize various compounds by direct condensation of an aromatic ring (for example, a benzene ring, an indole ring, a dicyano-substituted thiophene ring, a thiazole ring, or a pyrazine ring) with an NDI skeleton, and the resulting compounds are used for low-molecular organic semiconductors. However, such compounds are deteriorated in planarity and insufficient in extension of conjugation. Hence, the organic semiconductor device using such compounds cannot ensure satisfactory performance.

In the compounds discussed in PTL 2, thiophene rings extend from both sides of the naphthalene skeleton of NDI. Such compounds have a skeleton suitable for a macromolecular structure, and the exemplified low-molecular compounds still have insufficient semiconductor properties.

In a compound reported in PTL 3, a benzothiophene skeleton extends from one side of the naphthalene skeleton of NDI. PTL 3 utilizes this compound only as a crystallization inhibitor for an electrophotographic photoreceptor, and mentions nothing about its semiconductor properties.

Citation List

Patent Literature

PTL 1 CN 101885732 A
PTL 2 WO 2014/178415 A1
PTL 3 JP 5887323 B2

Non-Patent Literature

NPL 1 Chemical Communications, 2011, 47, pp. 10112-10114
NPL 2 Chemical Communications, 2011, 47, pp. 11504-11506
NPL 3 Chemistry of Materials, 2011, 23(5), pp. 1204-1215
NPL 4 Journal of Materials Chemistry C, 2013, 1, pp. 1087-1092
NPL 5 Organic & Biomolecular Chemistry, 2012, 10, pp. 6455-6468

Summary of Invention

Technical Problem

An object of the present invention is to provide a novel aromatic compound which has semiconductor properties represented by excellent carrier mobility, an organic semiconductor material containing the novel aromatic compound, a thin film forming composition containing the novel aromatic compound, an organic thin film containing the novel aromatic compound, and an organic semiconductor device containing the organic thin film.

Solution to Problem

Through intensive researches to achieve the above object, the inventors of the present invention have successfully developed a novel aromatic compound, and have found out that the novel aromatic compound has semiconductor properties represented by excellent carrier mobility. This finding has enabled an organic semiconductor material containing the novel aromatic compound, a thin film forming composition containing the novel aromatic compound, an organic thin film containing the novel aromatic compound, and an organic semiconductor device containing the organic thin film, leading to completion of the present invention.

Namely, the present invention relates to:

[1] an aromatic compound represented by General Formula (1),

[Chemical Formula 1]

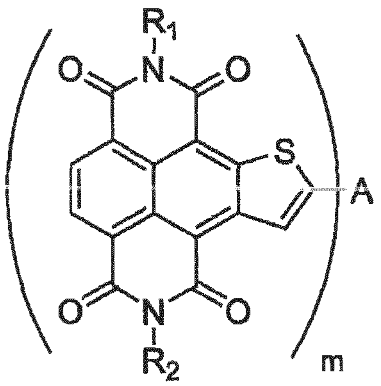

(1)

wherein each of $R_1$ and $R_2$ independently represents a hydrogen atom, a saturated hydrocarbon group having 1 to 30 carbon atoms, a saturated fluorohydrocarbon group having 1 to 30 carbon atoms, an aryl group, or a styryl group,
wherein the saturated hydrocarbon group having 1 to 30 carbon atoms, the saturated fluorohydrocarbon group having 1 to 30 carbon atoms, the aryl group, and the styryl group may have a substituent,
wherein m is an integer from 1 to 6,
wherein: when m is 1, A represents a hydrogen atom, a halogen atom, a hydrocarbon-trisubstituted silyl group, an aryl group, or a heteroaryl group; when m is 2, A represents a direct bond, or a divalent linking group having an aromatic ring or a heterocyclic ring; and when m is from 3 to 6, A represents a tri- to hexavalent linking group having an aromatic ring or a heterocyclic ring,
wherein, if the aromatic compound contains more than one $R_1$, $R_1$ may be identical to or different from each other; and if the aromatic compound contains more than one $R_2$, $R_2$ may be identical to or different from each other;

[2] the aromatic compound according to Item [1] above, wherein m is 2, and A represents a direct bond;
[3] the aromatic compound according to Item [1] above, wherein m is 2, and A represents a divalent linking group having an aromatic ring or a heterocyclic ring;
[4] the aromatic compound according to Item [1] above, wherein m is 3, and A represents a trivalent linking group having an aromatic ring or a heterocyclic ring;
[5] the aromatic compound according to Item [1] above, wherein m is 4, and A represents a tetravalent linking group having an aromatic ring or a heterocyclic ring;
[6] the aromatic compound according to Item [1] above, wherein m is 6, and A represents a hexavalent linking group having an aromatic ring or a heterocyclic ring;
[7] the aromatic compound according to any one of Items [1] to [6] above, wherein $R_1$ and $R_2$ represent an identical alkyl group having 1 to 30 carbon atoms;
[8] the aromatic compound according to any one of Items [1] to [6] above, wherein $R_1$ and $R_2$ represent an identical aryl group having 6 to 12 carbon atoms;
[9] an organic semiconductor material containing the aromatic compound according to any one of Items [1] to [8]

above;

[10] a thin film forming composition containing the aromatic compound according to any one of Items [1] to [8], and an organic solvent;

[11] an organic thin film containing the aromatic compound according to any one of Items [1] to [8];

[12] an organic semiconductor device containing the organic thin film according to Item [11] above;

[13] the organic semiconductor device according to Item [12], wherein the organic semiconductor device is an organic photoelectric transducer; and

[14] the organic semiconductor device according to Item [12] above, wherein the organic semiconductor device is an organic thin film transistor.

Advantageous Effects of Invention

An organic thin film containing the novel aromatic compound of the present invention represented by General Formula (1) can be used as a semiconductor layer of an organic semiconductor device, thereby enabling an organic semiconductor device which has excellent semiconductor properties such as higher photoelectric conversion performance and higher carrier mobility than a device using a conventional organic semiconductor material. Further, the aromatic compound of the present invention, wherein m is 1 and A is a halogen atom or a hydrocarbon-trisubstituted silyl group, has a thiophene ring which is condensed at only one side of the NDI skeleton and which has a reactive substituent in $\alpha$-position of the thiophene ring. This specific compound, which can be bonded with various polyvalent linking groups, is useful as an end cap for production of the aromatic compound of the present invention wherein m is two or greater.

Brief Description of Drawings

Figs. 1(a) to 1(f) are schematic views showing embodiments of an organic thin film transistor, as an organic semiconductor device according to the present invention.

Fig. 2 is a schematic view of an embodiment of an organic photoelectric transducer, as another organic semiconductor device according to the present invention.

Fig. 3 shows the gate voltage-drain current characteristics (transmission characteristics) of an organic thin film transistor obtained in Example 16 using the aromatic compound represented by Formula (23) in the present invention.

Fig. 4 is a graph showing current density-voltage characteristics of the organic photoelectric transducer according to the present invention, obtained in Example 17.

Fig. 5 is a graph showing current density-voltage characteristics of the organic photoelectric transducer according to the present invention, obtained in Example 18.

Fig. 6 is a graph showing current density-voltage characteristics of the organic photoelectric transducer according to the present invention, obtained in Example 19.

Description of Embodiments

[Aromatic Compound]

An aromatic compound according to the present invention has a structure represented by General Formula (1) as given above.

In General Formula (1), each of $R_1$ and $R_2$ represents a hydrogen atom, a saturated hydrocarbon group (an alkyl group or a cycloalkyl group) having 1 to 30 carbon atoms, a saturated fluorohydrocarbon group (a fluoroalkyl group or a fluorocycloalkyl group) having 1 to 30 carbon atoms, an aryl group, or a styryl group. The saturated hydrocarbon group having 1 to 30 carbon atoms, the saturated fluorohydrocarbon group having 1 to 30 carbon atoms, the aryl group, and the styryl group, represented by $R_1$ and $R_2$, may have a substituent. $R_1$ and $R_2$, which may be identical to or different from each other, are preferably identical to each other. If the compound includes more than one $R_1$ (when m is two or greater), $R_1$ may be identical to or different from each other. If the compound includes more than one $R_2$ (when m is two or greater), $R_2$ may be identical to or different from each other.

The saturated hydrocarbon group having 1 to 30 carbon atoms represented by $R_1$ and $R_2$ may be linear, branched, or annular, as long as having 1 to 30 carbon atoms. Preferably, each of $R_1$ and $R_2$ independently represents a linear or branched saturated hydrocarbon group, namely, an alkyl group. The linear saturated hydrocarbon group preferably has 1 to 16 carbon atoms, and more preferably has 1 to 12 carbon atoms. More specifically, the preferable linear saturated hydrocarbon group is a methyl group, an ethyl group, a hexyl group, an octyl group, a decanyl group, an undecanyl group, or the like. The branched saturated hydrocarbon group preferably has 6 to 30 carbon atoms, and

more preferably has 8 to 24 carbon atoms. More specifically, the preferable branched saturated hydrocarbon group is a 2-ethylhexyl group, a 3-ethylheptyl group, a 4-ethyloctyl group, a 2-butyloctyl group, a 3-butylnonyl group, a 4-butyldecyl group, a 2-hexyldecyl group, a 3-octylundecyl group, a 4-octyldodecyl group, a 2-octyldodecyl group, a 2-decyltetradecyl group, a 1-hexylheptyl group, or the like. The annular saturated hydrocarbon group (a cycloalkyl group) preferably has 5 to 10 carbon atoms, and more preferably has 5 or 6 carbon atoms. More specifically, the preferable annular saturated hydrocarbon group is a cyclopentyl group, a cyclohexyl group, a cycloheptyl group, a norbornyl group, a bicyclo[2,2,2]octyl group, an adamantyl group, or the like, among which a cyclopentyl group or a cyclohexyl group is more preferable. If the aromatic compound represented by General Formula (1) is dissolved for use in an organic solvent, the saturated hydrocarbon group is preferably branched.

The substituent that may be present in the saturated hydrocarbon group having 1 to 30 carbon atoms represented by $R_1$ and $R_2$ is not particularly limited, and may be, for example, a hydroxyl group, an alkoxy group, a cycloalkoxy group, an amino group, a cyano group, or the like. In the saturated hydrocarbon group having 1 to 30 carbon atoms represented by $R_1$ and $R_2$, the number and the position of the substituents are not particularly limited.

The saturated fluorohydrocarbon group having 1 to 30 carbon atoms represented by $R_1$ and $R_2$ is a substituent in which all or a part of the hydrogen atoms in the saturated hydrocarbon group having 1 to 30 carbon atoms are replaced by fluorine atoms.

The saturated fluorohydrocarbon group having 1 to 30 carbon atoms represented by $R_1$ and $R_2$ may be linear, branched, or annular, as long as having 1 to 30 carbon atoms. The preferable numbers of carbon atoms in the linear, branched, and annular saturated fluorohydrocarbon group are the same as the preferable numbers of carbon atoms in the saturated hydrocarbon group having 1 to 30 carbon atoms represented by $R_1$ and $R_2$ as specified above.

The substituent that may be present in the saturated fluorohydrocarbon group having 1 to 30 carbon atoms represented by $R_1$ and $R_2$ may be the same as the substituent that may be present in the saturated hydrocarbon group having 1 to 30 carbon atoms represented by $R_1$ and $R_2$. Just as the saturated hydrocarbon group having 1 to 30 carbon atoms, the number and the position of the substituents are not particularly limited.

The aryl group represented by $R_1$ and $R_2$ preferably has 6 to 12 carbon atoms. Specific examples of the aryl group represented by $R_1$ and $R_2$ include a phenyl group, a naphthyl group, and a biphenyl group, among which a phenyl group is preferred.

The substituent that may be present in the aryl group represented by $R_1$ and $R_2$ may be the above-mentioned saturated hydrocarbon group having 1 to 30 carbon atoms, or may be the same as the substituent that may be present in the saturated hydrocarbon group having 1 to 30 carbon atoms represented by $R_1$ and $R_2$. Just as the saturated hydrocarbon group having 1 to 30 carbon atoms, the number and the position of the substituents are not particularly limited. A preferred substituent is a saturated hydrocarbon group. In other words, the aryl group represented by $R_1$ and $R_2$ is preferably an aryl group, or an aryl group substituted with a saturated hydrocarbon (an alkyl aryl group or a cycloalkyl aryl group), and is more preferably a phenyl group, or a phenyl group substituted with a saturated hydrocarbon (an alkyl phenyl group or a cycloalkyl phenyl group).

The substituent that may be present in the styryl group represented by $R_1$ and $R_2$ may be the above-mentioned saturated hydrocarbon group having 1 to 30 carbon atoms, or may be the same as the substituent that may be present in the saturated hydrocarbon group having 1 to 30 carbon atoms represented by $R_1$ and $R_2$. Just as the saturated hydrocarbon group having 1 to 30 carbon atoms, the number and the position of the substituents are not particularly limited. A preferred substituent is a saturated hydrocarbon group. In other words, the styryl group represented by $R_1$ and $R_2$ is preferably a styryl group, or a styryl group substituted with a saturated hydrocarbon (an alkyl styryl group or a cycloalkyl styryl group).

Preferably, each of $R_1$ and $R_2$ in General Formula (1) is independently a saturated hydrocarbon group having 1 to 30 carbon atoms. More preferably, each of $R_1$ and $R_2$ is independently a linear saturated hydrocarbon group having 1 to 16 carbon atoms or a branched saturated hydrocarbon group having 6 to 30 carbon atoms. Further preferably, each of $R_1$ and $R_2$ is independently a linear saturated hydrocarbon group having 1 to 12 carbon atoms or a branched saturated hydrocarbon group having 8 to 24 carbon atoms. Particularly preferably, both of $R_1$ and $R_2$ are an identical linear saturated hydrocarbon group each having 1 to 12 carbon atoms or an identical branched saturated hydrocarbon group each having 8 to 24 carbon atoms. Also preferably, both of $R_1$ and $R_2$ in General Formula (1) are an identical alkyl group having 1 to 30 carbon atoms or an identical aryl group having 6 to 12 carbon atoms.

In General Formula (1), m is an integer from 1 to 6. When m is 1, A represents any of a hydrogen atom, a halogen atom, a hydrocarbon-trisubstituted silyl group (a silyl group substituted with three hydrocarbon groups, such as a trialkylsilyl group), an aryl group, or a heteroaryl group. When m is 2, A represents a direct bond, or a divalent linking group having an aromatic ring or a heterocyclic ring. When m is from 3 to 6, A represents a tri- to hexavalent linking group having an aromatic ring or a heterocyclic ring.

When m is 1, specific examples of the halogen atom represented by A include a fluorine atom, a chlorine atom, a bromine atom, and an iodine atom. When m is 1, a preferable halogen atom represented by A is a bromine atom or an iodine atom. When A represents a halogen atom, the aromatic compound of General Formula (1) can react with a

compound whose structure includes a potential part to be a linking group (to be described).

When m is 1, the hydrocarbon group in the hydrocarbon-trisubstituted silyl group represented by A is preferably a saturated hydrocarbon group, which usually has 1 to 10 carbon atoms and preferably has 1 to 6 carbon atoms. When m is 1, the hydrocarbon-trisubstituted silyl group represented by A may be, for example, a trimethylsilyl group, a triethylsilyl group, a tert-butyldimethylsilyl group, a triisopropylsilyl group, a tert-butyldiphenylsilyl group, etc.

When m is 1, the aryl group represented by A may be the same aryl group as represented by $R_1$ and $R_2$.

When m is 1, the heteroaryl group represented by A is an aryl group having at least one hetero atom (such as a sulfur atom, an oxygen atom, or a nitrogen atom) in the ring structure. Specific examples of the heteroaryl group include a pyridyl group, a pyrazyl group, a pyrimidyl group, a quinolyl group, an isoquinolyl group, a pyrrolyl group, a pyranyl group, a pyridonyl group, an indolenyl group, an imidazolyl group, a carbazolyl group, a thienyl group, a dithienyl group, a terthienyl group, a benzothienyl group, a thienothienyl group, a furyl group, a benzofuryl group, an anthraquinolyl group, an oxazolyl group, a thiazolyl group, etc.

General Formula (2) below represents a compound when m is 2 and A represents a direct bond. $R_1$ and $R_2$ in General Formula (2) have the same meaning as $R_1$ and $R_2$ in General Formula (1), and the compound contains more than one $R_1$ and more than one $R_2$.

[Chemical Formula 2]

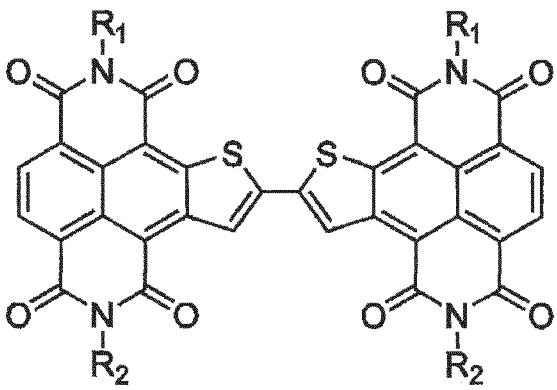

(2)

When m is from 2 to 6 and A is not a direct bond, the linking group having an aromatic ring or a heterocyclic ring represented by A has a valence corresponding to the integer m. Namely, the linking group having an aromatic ring or a heterocyclic ring represented by A is divalent when m is 2, trivalent when m is 3, tetravalent when m is 4, pentavalent when m is 5, and hexavalent when m is 6. The integer m is preferably 2, 3, 4, or 6, which means that the linking group is preferably divalent, trivalent, tetravalent, or hexavalent.

Specific examples of the divalent, trivalent, tetravalent, and hexavalent linking groups are given below.

The divalent linking groups include, for example, divalent aryl groups and divalent heteroaryl groups, as specifically shown below. Each divalent linking group may have a substituent, various examples of which are given above as the substituents that may be present in the saturated hydrocarbon group having 1 to 30 carbon atoms represented by $R_1$ and $R_2$.

[Chemical Formula 3]

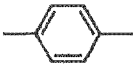
A2-1

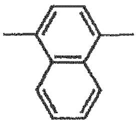
A2-2

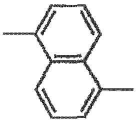
A2-3

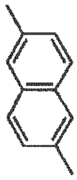
A2-4

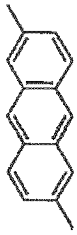
A2-5

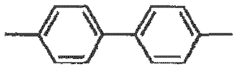
A2-6

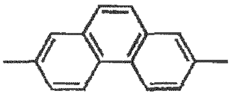
A2-7

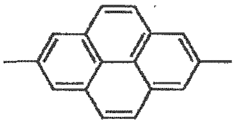
A2-8

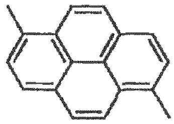
A2-9

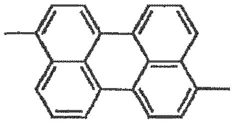
A2-10

[Chemical Formula 4]

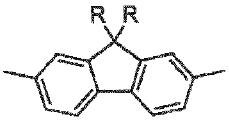
A2-11

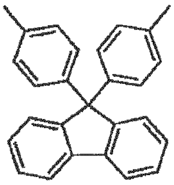
A2-12

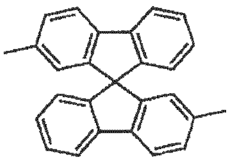
A2-13

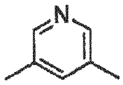
A2-14

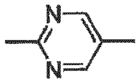
A2-15

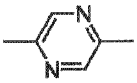
A2-16

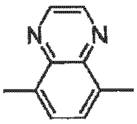
A2-17

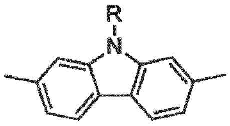
A2-18

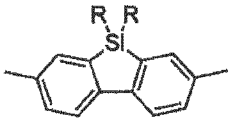
A2-19

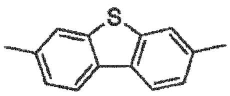
A2-20

[Chemical Formula 5]

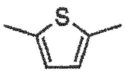
A2-21

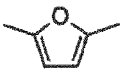
A2-22

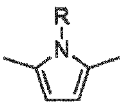
A2-23

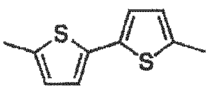
A2-24

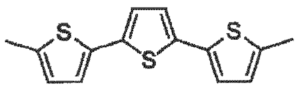
A2-25

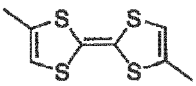
A2-26

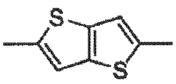
A2-27

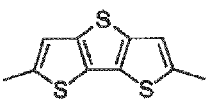
A2-28

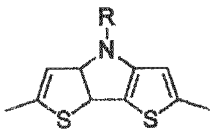
A2-29

[Chemical Formula 6]

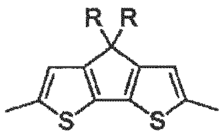
A2-30

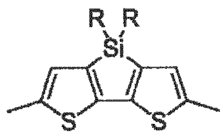
A2-31

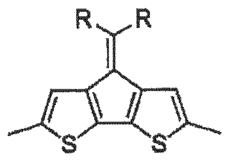
A2-32

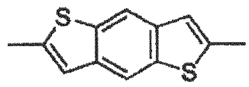
A2-33

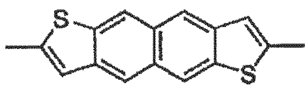
A2-34

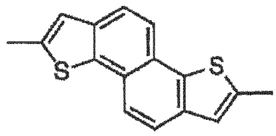
A2-35

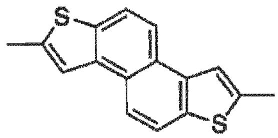
A2-36

[Chemical Formula 7]

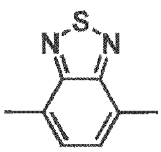
A2-37

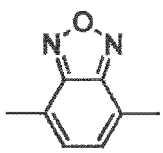
A2-38

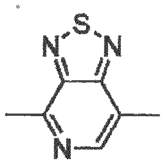
A2-39

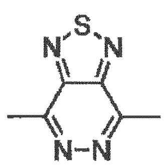
A2-40

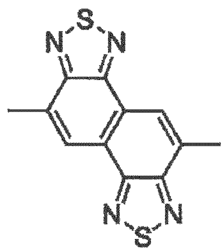
A2-41

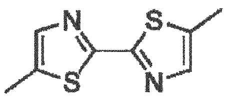
A2-42

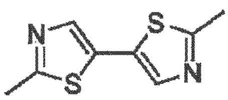
A2-43

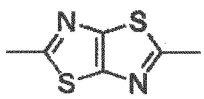
A2-44

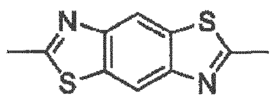
A2-45

[Chemical Formula 8]

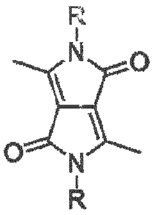
A2-46

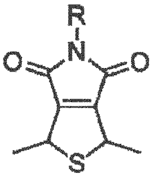
A2-47

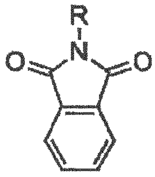
A2-48

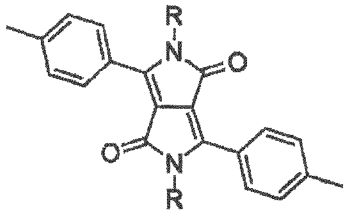
A2-49

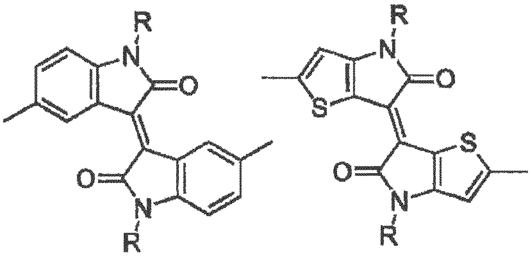
A2-50

A2-51

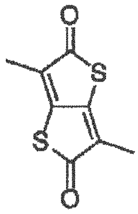
A2-52

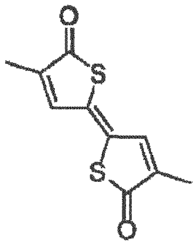
A2-53

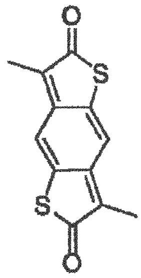
A2-54

[Chemical Formula 9]

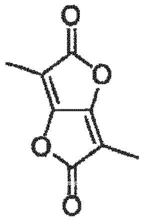
A2-55

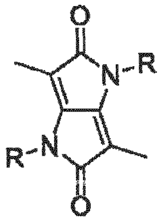
A2-56

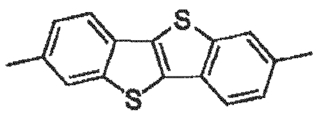
A2-57

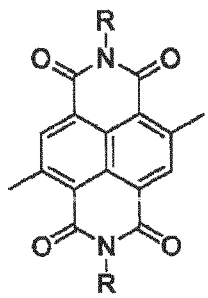
A2-58

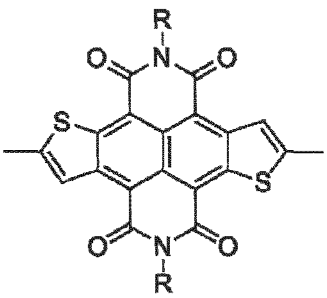
A2-59

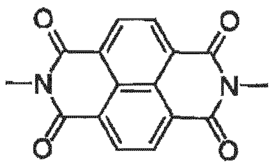
A2-60

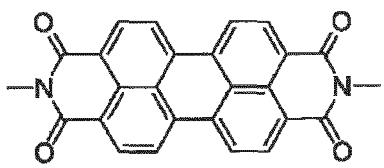
A2-61

In Formulas A2-11, A2-18, A2-19, A2-23, A2-29, A2-30, A2-31, A2-32, A2-46, A2-47, A2-48, A2-49, A2-50, A2-51, A2-56, A2-58, and A2-59, R is a hydrogen atom, a saturated hydrocarbon group having 1 to 30 carbon atoms, a saturated fluorohydrocarbon group having 1 to 30 carbon atoms, an aryl group, or a styryl group. The saturated hydrocarbon group having 1 to 30 carbon atoms, the saturated fluorohydrocarbon group having 1 to 30 carbon atoms, the aryl group, and the styryl group, represented by R, may have a substituent. If each of the linking groups represented by the above Formulas contains two Rs, the two Rs may be identical to or different from each other.

The saturated hydrocarbon group having 1 to 30 carbon atoms represented by R may be linear, branched, or

annular, as long as having 1 to 30 carbon atoms. Preferably, each R independently represents a linear or branched saturated hydrocarbon group, namely, an alkyl group. The linear saturated hydrocarbon group preferably has 1 to 16 carbon atoms, and more preferably has 1 to 12 carbon atoms. More specifically, the preferable linear saturated hydrocarbon group is a methyl group, an ethyl group, a hexyl group, an octyl group, a decanyl group, an undecanyl group, or the like. The branched saturated hydrocarbon group preferably has 6 to 30 carbon atoms, and more preferably has 8 to 24 carbon atoms. More specifically, the preferable branched saturated hydrocarbon group is a 2-ethylhexyl group, a 3-ethylheptyl group, a 4-ethyloctyl group, a 2-butyloctyl group, a 3-butylnonyl group, a 4-butyldecyl group, a 2-hexyldecyl group, a 3-octylundecyl group, a 4-octyldodecyl group, a 2-octyldodecyl group, a 2-decyltetradecyl group, a 1-hexylheptyl group, or the like. The annular saturated hydrocarbon group preferably has 5 to 10 carbon atoms, and more preferably has 5 or 6 carbon atoms. More specifically, the preferable annular saturated hydrocarbon group is a cyclopentyl group, a cyclohexyl group, a cycloheptyl group, a norbornyl group, a bicyclo[2,2,2]octyl group, an adamantyl group, or the like, among which a cyclopentyl group or a cyclohexyl group is more preferable. If the aromatic compound represented by General Formula (1) is dissolved for use in an organic solvent, the saturated hydrocarbon group is preferably branched.

The substituent that may be present in the saturated hydrocarbon group having 1 to 30 carbon atoms represented by R is not particularly limited and may be, for example, a hydroxyl group, an alkoxy group, a cycloalkoxy group, an amino group, a cyano group, or the like. In the saturated hydrocarbon group having 1 to 30 carbon atoms represented by R, the number and the position of the substituents are not particularly limited.

The saturated fluorohydrocarbon group having 1 to 30 carbon atoms represented by R is a substituent in which all or a part of the hydrogen atoms in the saturated hydrocarbon group having 1 to 30 carbon atoms are replaced by fluorine atoms.

The saturated fluorohydrocarbon group having 1 to 30 carbon atoms represented by R may be linear, branched, or annular, as long as having 1 to 30 carbon atoms. The preferable numbers of carbon atoms in the linear, branched, and annular saturated fluorohydrocarbon group are the same as the preferable numbers of carbon atoms in the saturated hydrocarbon group having 1 to 30 carbon atoms represented by R as specified above.

The substituent that may be present in the saturated fluorohydrocarbon group having 1 to 30 carbon atoms represented by R may be the same as the substituent that may be present in the saturated hydrocarbon group having 1 to 30 carbon atoms represented by R. Just as the saturated hydrocarbon group having 1 to 30 carbon atoms, the number and the position of the substituents are not particularly limited.

The aryl group represented by R preferably has 6 to 12 carbon atoms. Specific examples of the aryl group represented by R include a phenyl group, a naphthyl group, and a biphenyl group, among which a phenyl group is preferred.

The substituent that may be present in the aryl group represented by R may be the above-mentioned saturated hydrocarbon group having 1 to 30 carbon atoms, or may be the same as the substituent that may be present in the saturated hydrocarbon group having 1 to 30 carbon atoms represented by R. Just as the saturated hydrocarbon group having 1 to 30 carbon atoms, the number and the position of the substituents are not particularly limited. A preferred substituent is a saturated hydrocarbon group. In other words, the aryl group represented by R is preferably an aryl group, or an aryl group substituted with a saturated hydrocarbon, and is more preferably a phenyl group, or a phenyl group substituted with a saturated hydrocarbon.

The substituent that may be present in the styryl group represented by R may be the above-mentioned saturated hydrocarbon group having 1 to 30 carbon atoms, or may be the same as the substituent that may be present in the saturated hydrocarbon group having 1 to 30 carbon atoms represented by R. Just as the saturated hydrocarbon group having 1 to 30 carbon atoms, the number and the position of the substituents are not particularly limited. A preferred substituent is a saturated hydrocarbon group. In other words, the styryl group represented by R is preferably a styryl group, or a styryl group substituted with a saturated hydrocarbon.

Preferably, R is a saturated hydrocarbon group having 1 to 30 carbon atoms. More preferably, R is a linear saturated hydrocarbon group having 1 to 16 carbon atoms or a branched saturated hydrocarbon group having 6 to 30 carbon atoms. Further preferably, R is a linear saturated hydrocarbon group having 1 to 12 carbon atoms or a branched saturated hydrocarbon group having 8 to 24 carbon atoms. In the linking groups represented by the above Formulas and having two Rs, it is particularly preferable that the two Rs are an identical linear saturated hydrocarbon group having 1 to 12 carbon atoms or an identical branched saturated hydrocarbon group having 8 to 24 carbon atoms. Further, in the linking groups represented by the above Formulas and having two Rs, the two Rs are preferably an identical alkyl group having 1 to 30 carbon atoms or an identical aryl group having 6 to 12 carbon atoms.

Examples of the trivalent linking groups are given below. Each of these trivalent linking groups may have a substituent, various examples of which are given above as the substituents that may be present in the saturated hydrocarbon group having 1 to 30 carbon atoms represented by $R_1$ and $R_2$.

[Chemical Formula 10]

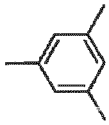

A3-1

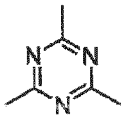

A3-2

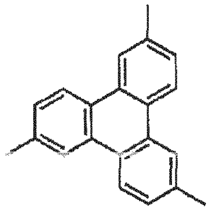

A3-3

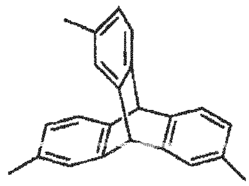

A3-4

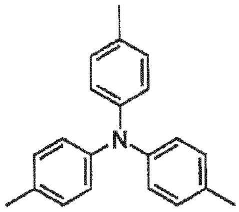

A3-5

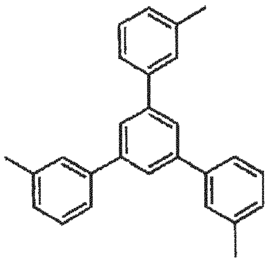

A3-6

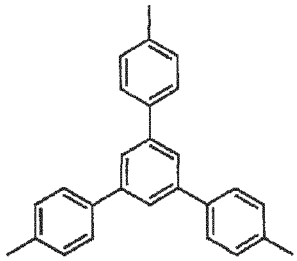

A3-7

Examples of the tetravalent linking groups are given below. Each of these tetravalent linking groups may have a substituent, various examples of which are given above as the substituents that may be present in the saturated hydrocarbon group having 1 to 30 carbon atoms represented by $R_1$ and $R_2$.

[Chemical Formula 11]

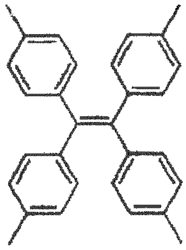
A4-1

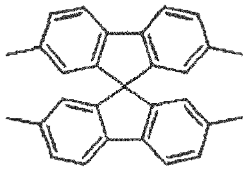
A4-2

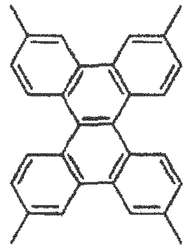
A4-3

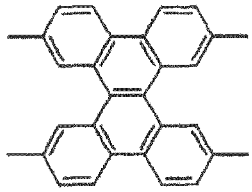
A4-4

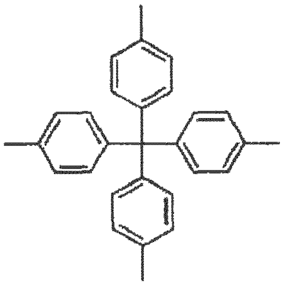
A4-5

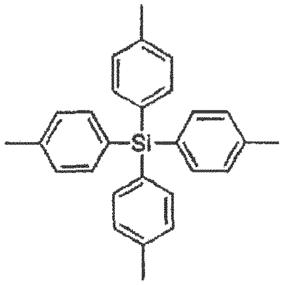
A4-6

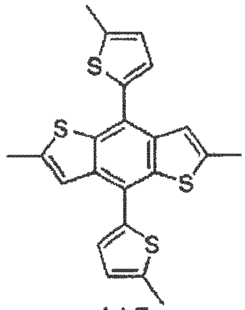
A4-7

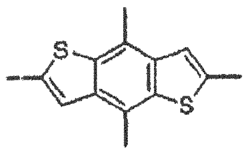
A4-8

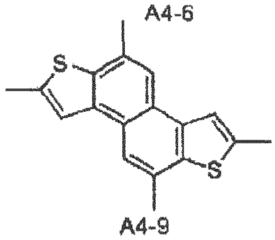
A4-9

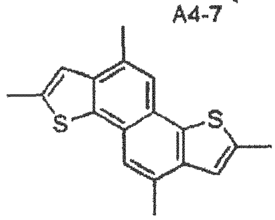
A4-10

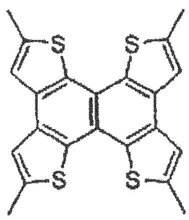
A4-11

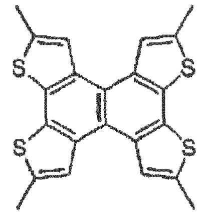
A4-12

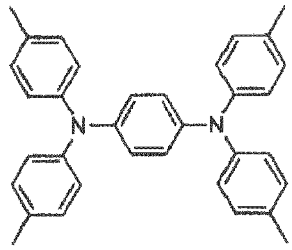
A4-13

Examples of the hexavalent linking groups are given below. This hexavalent linking group may have a substituent, various examples of which are given above as the substituents that may be present in the saturated hydrocarbon group having 1 to 30 carbon atoms represented by $R_1$ and $R_2$.

[Chemical Formula 12]

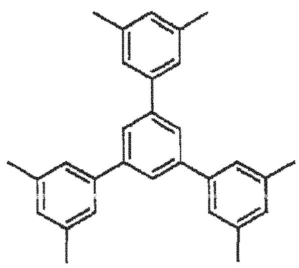
A6-1

In the present invention, the divalent linking group represented by A encompasses, for example, any of the above trivalent linking groups in which one of the three linking hands is bonded to a substituent whose partial structure

is not the one shown in the parentheses in General Formula (1). The same applies to the tetra- to hexavalent linking groups.

Among the specific examples of the linking groups given above, preferable divalent linking groups are A2-1, A2-2, A2-6, A2-7, A2-8, A2-10, A2-11, A2-12, A2-13, A2-15, A2-18, A2-25, A2-26, A2-27, A2-33, A2-35, A2-36, A2-37, A2-44, A2-45, A2-46, A2-47, A2-52, A2-57, A2-58, and A2-59; preferable trivalent linking groups are A3-1, A3-3, A3-5, and A3-7; preferable tetravalent linking groups are A4-2, A4-3, A4-5, A4-6, A4-7, A4-9, A4-10, and A4-13; and a preferable hexavalent linking group is A6-1. Of these preferable linking groups, A2-1, A2-7, A2-11, A2-13, A2-18, A2-24, A2-25, A2-27, A2-35, A2-36, A2-37, A2-44, A2-57, A2-58, A2-59, A3-1, A3-5, A3-7, A4-2, A4-6, A4-9, A4-13, and A6-1 are more preferable.

When m is from 2 to 6 and A is not a direct bond, the di- to hexavalent linking groups each having an aromatic ring or a heterocyclic ring represented by A encompass not only the linking groups of the above specific examples but also linking groups each having a substituent bonded to an aromatic ring or a heterocyclic ring. For example, any of the linking groups of the above specific examples each having an aromatic ring or a heterocyclic ring may further have a substituent such as an alkylene group bonded to the aromatic ring or the heterocyclic ring.

The aromatic compound represented by General Formula (1) can be synthesized, for example, in a known manner as disclosed in PTL 2, etc. by a process represented by reaction formulas below. In the following reaction formulas, $R_1$, $R_2$, A, and m have the same meaning as $R_1$, $R_2$, A, and m in General Formula (1).

[Chemical Formula 13]

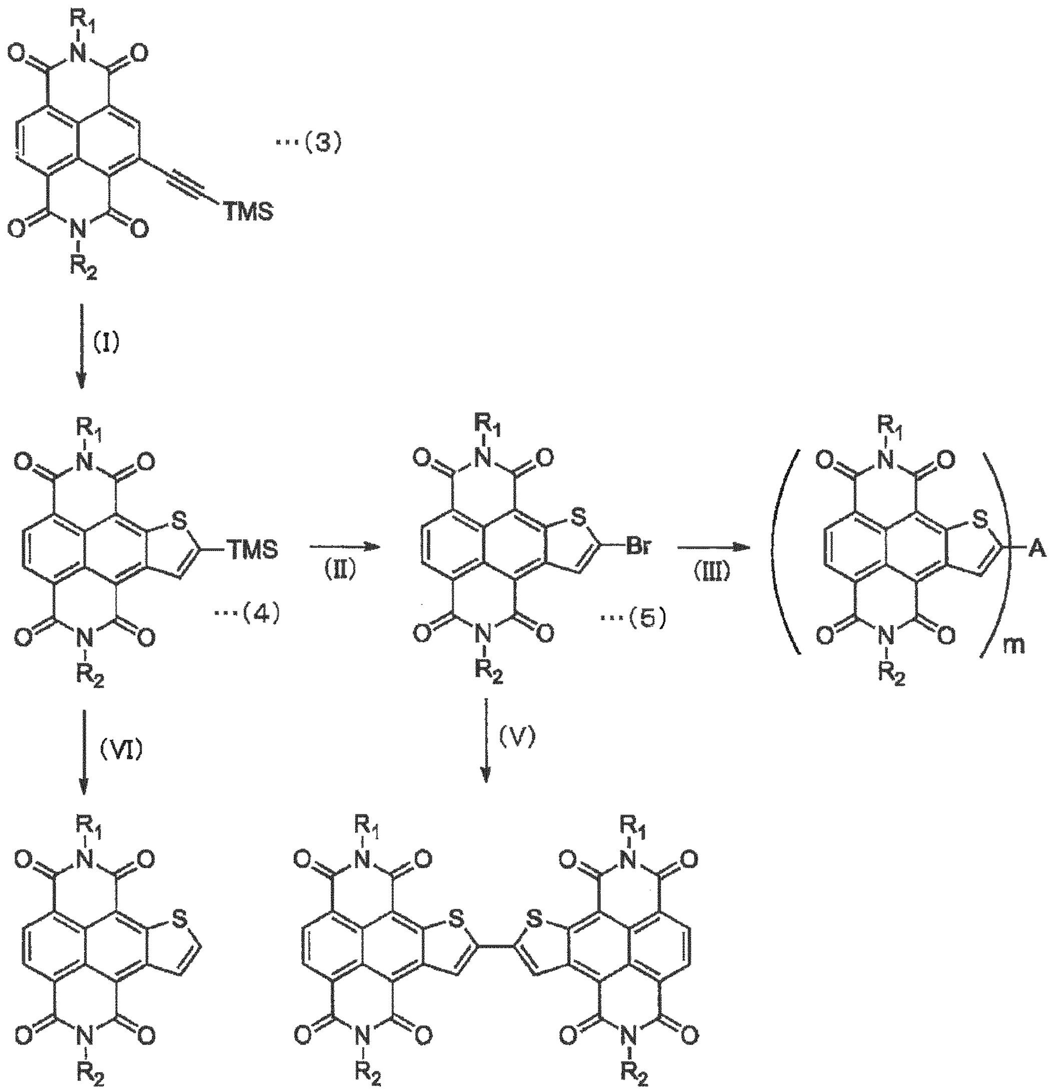


As the reaction (I) in the above reaction formulas, a compound shown by General Formula (3) is allowed to react with a sulfide salt. This reaction causes a cyclization reaction, causing condensation of a thiophene ring on one side of the naphthalene ring in the compound shown by General Formula (3), and thereby giving a compound shown

by General Formula (4). The sulfide salt is preferably a metal sulfide, and is more preferably an alkali metal sulfide. Examples of the alkali metal sulfide include sodium sulfide nonahydrate, sodium sulfide pentahydrate, anhydrous sodium sulfide, sodium hydrogen sulfide hydrate, etc. The trimethylsilyl group (TMS) in the compound represented by General Formula (3) may be replaced by a different hydrocarbon-trisubstituted silyl group, for example, a triethylsilyl group, a tert-butyldimethylsilyl group, a triisopropylsilyl group, a tert-butyldiphenylsilyl group, etc.

As the reaction (II) in the above reaction formulas, the compound shown by General Formula (4) is allowed to react with a brominating agent. The brominating agent may be, for example, bromine. The brominating agent may be replaced by a different halogenating agent (e.g. an iodinating agent such as iodine or iodine monochrolide). If the brominating agent is replaced by an iodinating agent, the reaction product is a compound represented by General Formula (5) in which the bromo group is replaced by the iodo group.

As the reaction (III) in the above reaction formulas, the compound represented by General Formula (5) is subjected to a coupling reaction, in the presence of a transition metal catalyst such as a palladium catalyst (e.g. tetrakis(triphenylphosphine)palladium), with an organometallic compound of the following Formula,

A(X)m

wherein X represents a metal-containing group such as a trialkyltin group (e.g. a trimethyltin group), a boronic acid group, and a boronate ester group (e.g. a boronic acid pinacol ester group).

As the desilylation reaction (IV) in the above reaction formulas, the compound represented by General Formula (4) is allowed to react with an acid, a base, or a fluoride. The fluoride may be, for example, tetra-n-butylammonium fluoride.

As the reaction (V) in the above reaction formulas, the compound represented by General Formula (5) is allowed to react in the presence of a transition metal catalyst such as a palladium catalyst (e.g. tetrakis(triphenylphosphine)palladium) and a trialkyltin forming agent such as bis(trimethyltin).

An aromatic compound represented by General Formula (1) wherein m is 1 and A is a halogen atom may be allowed to react, by Suzuki coupling, with an aromatic compound represented by General Formula (1) wherein m is 1 and A is an aryl group or a heteroaryl group. This coupling reaction can give an aromatic compound represented by General Formula (1) wherein m is 2 and A is a divalent aryl group or a divalent heteroaryl group. Specific examples of the aryl group and the heteroaryl group which can react with a halogen atom include a phenyl group, a biphenyl group, a naphthyl group, an anthryl group, a phenanthryl group, a pyrenyl group, a benzopyrenyl group, a fluorenyl group, an indenyl group, a pyridyl group, a pyrazyl group, a pyrimidyl group, a quinolyl group, an isoquinolyl group, a pyrrolyl group, a pyranyl group, a pyridonyl group, an indolenyl group, an imidazolyl group, a carbazolyl group, a thienyl group, a dithienyl group, a terthienyl group, a benzothienyl group, a thienothienyl group, a furyl group, a benzofuryl group, an anthraquinolyl group, an oxazolyl group, and a thiazolyl group. A preferable example is a phenyl group, a biphenyl group, a phenanthryl group, a thienyl group, a dithienyl group, a fluorenyl group, or a pyrazyl group. Each of these groups may have the above-mentioned substituent such as a saturated hydrocarbon group.

The method for purifying the aromatic compound represented by General Formula (1) is not particularly limited and may be seleted from known methods such as recrystallization, column chromatography, and vacuum sublimation. These methods can be combined as required.

[Organic Semiconductor Material]

An organic semiconductor material according to the present invention contains the aromatic compound represented by General Formula (1). The organic semiconductor material according to the present invention is suitable as a material for an organic thin film in organic electronic devices such as organic EL (electroluminescence) devices, organic photovoltaic cells, organic photoelectric transducers, and organic thin film transistors.

[Thin Film Forming Composition]

A thin film forming composition according to the present invention contains the aromatic compound represented by General Formula (1) and an organic solvent. Generally, the aromatic compound represented by General Formula (1) or an organic semiconductor material containing this aromatic compound is dissolved or dispersed in an organic solvent. The organic solvent may be a single organic solvent or a mixture of organic solvents.

In the thin film forming composition, the content of the aromatic compound represented by General Formula (1) may vary depending on the species of the organic solvent and the thickness of the thin film to be formed. Relative to 100 parts by mass of the organic solvent, the content of the aromatic compound is usually from 0.1 to 5 parts by mass, and is preferably from 0.3 to 5 parts by mass. The thin film forming composition according to the present invention simply needs to contain the aromatic compound represented by General Formula (1) as dissolved or dispersed in the solvent. Having said that, a preferable thin film forming composition is a homogeneous solution in which the aromatic compound

represented by General Formula (1) is dissolved uniformly.

The organic solvents for the thin film forming composition may be halogenated organic solvents such as chloroform, dichloromethane, chlorobenzene, dichlorobenzene, and 1,2,4-trichlorobenzene, but are preferably halogen-free organic solvents. Preferable halogen-free organic solvents include aromatic hydrocarbons such as benzene, toluene, xylene, mesitylene, ethylbenzene, tetrahydronaphthalene, and cyclohexylbenzene; ethers such as diethyl ether, tetrahydrofuran, anisole, phenetole, and butoxybenzene; amides such as dimethylacetamide, dimethylformamide, and N-methylpyrrolidone; ketones such as acetone, methyl ethyl ketone, methyl isobutyl ketone, cyclopentanone, and cyclohexanone; nitriles such as acetonitrile, propionitrile, and benzonitrile; alcohols such as methanol, ethanol, isopropanol, butanol, and cyclohexanol; esters such as ethyl acetate, butyl acetate, ethyl benzoate, and diethyl carbonate; hydrocarbons such as hexane, octane, decane, cyclohexane, and decalin.

In addition to the aromatic compound represented by General Formula (1) and the organic solvent as mentioned above, the thin film forming composition according to the present invention may further contain an additive, as required, in order to improve characteristics of the organic semiconductor device, to acquire a desired absorption band or other characteristics, or to achieve other objects. Such an additive is not particularly limited unless inhibiting the semiconductor function of the aromatic compound represented by General Formula (1) above. Examples of the additive include a semiconducting material having a different structure, an insulating material, a surfactant for rheology control, a thickening agent, a dopant for controlling the carrier injection and the carrier amount, etc. The additive may be high-molecular or low-molecular unless inhibiting the stability of the composition. The content of the additive, which varies with its purpose and thus cannot be generalized, is preferably less than the content of the aromatic compound represented by General Formula (1).

[Organic Thin Film]

The following description concerns an organic thin film according to the present invention. An organic thin film can be produced with use of the organic semiconductor material which contains the aromatic compound represented by General Formula (1) according to the present invention. The organic thin film according to the present invention contains at least one species of aromatic compound of General Formula (1), and may contain more than one species of aromatic compound represented by General Formula (1) according to the present invention or may contain a mixture of the aromatic compound represented by General Formula (1) according to the present invention and another functional material. Examples of the funtional material include low- or high-molecular, p-type, n-type, or ambipolar semiconductors, insulating polymers, and dopants. The functional material may be suitably selected in consideration of the intended use of the organic thin film and the organic semiconductor device using the organic thin film. The thickness of the organic thin film, which is different depending on its intended use, is usually from 1 nm to 10 $\mu$m, preferably from 5 nm to 3 $\mu$m, and more preferably from 10 nm to 1 pm.

The methods for forming the organic thin film include dry processes (e.g. vapor deposition and sputtering) or various solution processes, and may be suitably selected in consideration of physical properties (e.g. solubility, sublimation property) of the aromatic compound represented by General Formula (1). The solution processes include, for example, spin coating, drop casting, dip coating, spraying, bar coating, die coating, slit coating, pen process, curtain coating, flexography, relief printing, offset printing, dry offset printing, lithographic printing, gravure printing, screen printing, stencil printing, ink jet printing, microcontact printing, and combinations of these techniques. These solution processes can be conducted with use of the thin film forming composition according to the present invention in which the aromatic compound represented by General Formula (1) is dissolved. A preferable dry process for formation of the organic thin film is vapor deposition by resistive heating. A preferable solution process for formation of the organic thin film is spin coating, die coating, slit coating, offset printing, or ink jet printing. In a preferable solution process, a solution is applied or printed by any of the above-mentioned solution processes, and the organic solvent is allowed to evaporate to give the organic thin film.

For stable formation of an organic thin film, the environments on the film-forming surface (the surface on which an organic thin film is formed) such as surface energy and surface temperature during the film formation is important. Such environments may even change the condition of the organic thin film and the characteristics of the organic semiconductor device. For example, an improper surface energy on the film-forming surface may hamper formation of a continuous film due to defects such as cissing on the film-forming surface or may cause other problems. The forming temperature of the organic thin film or the drying temperature of the organic solvent or the like, the postprocessing (heat treatment) temperature after the thin film formation, and other like conditions can alleviate distortion in the organic thin film generated during the thin film formation, can reduce pinholes and the like, and can control the arrangement and orientation in the organic thin film. For these and other reasons, adjustment of the surface environments contributes to improvement and stabilization of the characteristics of the organic semiconductor device using the organic thin film. As the heat treatment, the substrate is heated after the organic thin film is formed. The heat treatment temperature is not particularly limited, but is usually from room temperature to about 200°C, preferably from 40 to 150°C, and more preferably

from 50 to 120°C. The heat treatment period is not particularly limited, but is usually from 10 seconds to 24 hours, preferably from about 30 seconds to about 3 hours. The heat treatment may be conducted in an air atmosphere but may be also conducted in an inert (e.g. nitrogen or argon) atmosphere. Additionally, the shape of the organic thin film is controllable by solvent vapor.

[Organic Semiconductor Device]

Next, an organic semiconductor device according to the present invention is described. An organic semiconductor device according to the present invention contains, as an organic semiconductor layer, the organic thin film containing at least one species of aromatic compound represented by General Formula (1) above. Examples of the organic semiconductor device include organic thin film transistors, organic EL (electroluminescence) devices (e.g. color organic EL devices), organic photoelectric transducers, diodes, capacitors, and other various devices.

The organic semiconductor device according to the present invention, which employs the aromatic compound represented by General Formula (1) as an organic semiconductor material, can be produced by a relatively low-temperature process. Hence, the substrate for constituting the organic semiconductor device can be a flexible substrate (e.g. a plastic plate and a plastic film) that was unsuitable in a high-temperature condition. Eventually, it is possible to produce a light-weight, highly flexible, break-proof organic semiconductor device.

[Organic Thin Film Transistor]

Next, an organic thin film transistor is described as an embodiment of the organic semiconductor device according to the present invention. An organic thin film transistor has a semiconductor layer made of an organic thin film and two electrodes (a source electrode and a drain electrode) in contact with the semiconductor layer, and controls electric current flowing across the electrodes by the voltage applied to another electrode called gate electrode.

A common structure for the organic thin film transistor is to insulate the gate electrode by an insulating film (Metal-Insulator-Semiconductor; MIS structure). A MIS structure having a metal oxide insulating film is called MOS (Metal-Oxide-Semiconductor) structure. Another structure for the thin film transistor is to provide the gate electrode on the semiconductor thin film via a Schottky barrier (i.e. MES structure). Having said that, the MIS structure is more frequent in the organic thin film transistor.

Hereinafter, the organic thin film transistor is described in greater detail, with reference to several exemplary embodiments shown in Fig. 1. However, the present invention should not be limited to such structures.

Figs. 1(a)-1(f) show organic thin film transistors 10A-10F as the exemplary embodiments. Each of these organic thin film transistors has a source electrode 1, a semiconductor layer 2, a drain electrode 3, an insulator layer 4, and a gate electrode 5. Each of the organic thin film transistors 10A-10D and 10F further has a substrate 6. The arrangement of these layers and electrodes can be suitably selected in consideration of the intended use of the organic thin film transistors. The organic thin film transistors 10A-10D and 10F conduct current in a direction parallel to the substrate 6, hence called lateral transistors. The organic thin film transistor 10A, called bottom-contact bottom-gate structure, is composed of a substrate 6, a gate electrode 5 formed on the substrate 6, an insulator layer 4 formed on the gate electrode 5, a source electrode 1 and a drain electrode 3 formed on the insulator layer 4, and a semiconductor layer 2 formed at the top. The organic thin film transistor 10B, called top-contact bottom-gate structure, is composed of a substrate 6, a gate electrode 5 formed on the substrate 6, an insulator layer 4 formed on the gate electrode 5, a semiconductor layer 2 formed on the insulator layer 4, and a source electrode 1 and a drain electrode 3 formed the semiconductor layer 2. The organic thin film transistor 10C, called top-contact top-gate structure, is composed of a substrate 6, a semiconductor layer 2 formed on the substrate 6, a source electrode 1 and a drain electrode 3 formed on the semiconductor layer 2, an insulator layer 4 formed on the electrodes 1, 3 and the semiconductor layer 2, and a gate electrode 5 formed on the insulator layer 4. The organic thin film transistor 10D, called top/bottom-contact bottom-gate structure, is composed of a substrate 6, a gate electrode 5 formed on the substrate 6, an insulator layer 4 formed on the gate electrode 5, a source electrode 1 formed on the insulator layer 4, a semiconductor layer 2 formed on the source electrode 1 and the insulator layer 4, and a drain electrode 3 formed on the semiconductor layer 2. The organic thin film transistor 10F, called bottom-contact top-gate structure, is composed of a substrate 6, a source electrode 1 and a drain electrode 3 formed on the substrate 6, a semiconductor layer 2 formed on the electrodes 1, 3 and the substrate 6, an insulator layer 4 formed on the semiconductor layer 2, and a gate electrode 5 formed on the insulator layer 4.

The organic thin film transistor 10E has a vertical structure, called static induction transistor (SIT). The SIT conducts electric current in a planar manner, and allows simultaneous movement of a large amount of carriers 8. Since a source electrode 1 and a drain electrode 3 are vertically opposed, the SIT has a smaller distance between the electrodes and a high response speed. For this reason, the SIT is advantageous for conduction of a large amount of current, high-speed switching, and other like applications. A substrate, omitted in Fig. 1(e), is usually provided on the outer side of the source electrode 1 or the drain electrode 3 in Fig. 1(e). The organic thin film according to the present invention can

serve as the semiconductor layer 2 in Fig. 1.

[Organic Photoelectric Transducer]

Next, an organic photoelectric transducer is described as another embodiment of the organic semiconductor device according to the present invention. The organic photoelectric transducer has an upper electrode and a bottom electrode opposed to each other, and a photoelectric conversion film made of the organic thin film and disposed between the two opposed electrodes as a photoelectric conversion unit. From above one or both of the electrodes, light is incident on the photoelectric conversion unit. The photoelectric conversion unit generates electrons and holes corresponding to the amount of incident light. Examples of the organic photoelectric transducer include: a photovoltaic cell which transports the electrons and holes generated in the photoelectric conversion unit and which collects the electrons and holes at the electrodes to obtain the electromotive force; an imaging device for forming an image, wherein the semiconductor reads a signal which corresponds to the electric charge of the photoelectric conversion unit and which indicates the amount of incident light in accordance with the absorption wavelength of the photoelectric conversion unit; and the like.

Fig. 2 shows an exemplary embodiment of the organic photoelectric transducer.

The organic photoelectric transducer according to the exemplary embodiment shown in Fig. 2 has a substrate 15, a bottom electrode 14 formed on the substrate 15, a photoelectric conversion unit 13 formed on the bottom electrode 14, an upper electrode 12 formed on the photoelectric conversion unit 13, and an insulation unit 11 formed on the upper electrode 12. The light incident on the organic photoelectric transducer may come from above or below the organic photoelectric transducer as long as the components of the organic photoelectric transducer other than the photoelectric conversion unit 13 do not excessively obstruct the light at the absorption wavelength of the photoelectric conversion unit 13. In many cases, the photoelectric conversion unit 13 is composed of a plurality of layers including a photoelectric conversion layer, an electron transport layer, a hole transport layer, an electron blocking layer, a hole blocking layer, etc., but should not be limited thereto. Each of the constitutive layers of the photoelectric conversion unit 13 (i.e. a photoelectric conversion layer, an electron transport layer, a hole transport layer, an electron blocking layer, and a hole blocking layer) is a thin film made of a p-type organic semiconductor, a thin film made of an n-type organic semiconductor, or a combined thin film of these organic semiconductors (bulk heterostructure). Each constitutive layer is made of a single thin film or a plurality of thin films. The organic thin film according to the present invention can be mainly utilized as the photoelectric conversion unit 13 in Fig. 2.

[Examples]

Hereinafter, the present invention is described in greater detail by way of Examples in which, for example, the aromatic compounds represented by General Formula (1) were synthesized. However, such Examples are not intended to limit the present invention.

As the solvents in the following Examples, solvents obtained by anhydrous distillation were employed for reactions and measurements under inert gas atmosphere, and commercial first-grade or special-grade solvents were employed for the other reactions and operations. The reagents for the reactions were purified by anhydrous distillation or the like, if necessary. Otherwise, commercial first-grade or special-grade reagents were employed without special treatment.

Regarding the aromatic compounds synthesized in the following Examples, the proton nuclear magnetic resonance spectrum (hereinafter "$^1$H-NMR") was obtained by measurement of the chemical shift $\sigma$ (ppm), etc., using a nuclear magnetic resonance spectrometer (Model "LAMBDA 400", manufactured by JEOL Ltd.).

[Example 1] (Synthesis of an aromatic compound of the present invention, represented by Formula (11) below)

In a nitrogen atmosphere, N,N'-dioctyl-2-[2-(trimethylsilyl)ethynyl]-1,4,5,8-naphthalenetetraca rboxylic acid diimide (4 mmol), sodium sulfide nonahydrate (24 mmol), acetic acid (8 mL), and 2-methoxyethanol (400 mL) were fed into a one-liter eggplant-shaped recovery flask and stirred at 60°C for 12 hours. The reaction liquid was cooled down to room temperature and poured into water (400 mL). The solid deposit was filtered off. The filtered solid was rinsed first with water and then with methanol, and purified by silica gel column chromatography. Thus obtained was an orange, solid, aromatic compound represented by Formula (11) below.

[Chemical Formula 14]

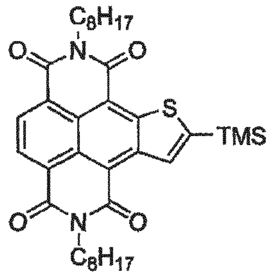

(11)

The yield of the aromatic compound represented by Formula (11) was 65%,

The nuclear magnetic resonance spectrum of the aromatic compound represented by Formula (11) is given below.
$^1$H-NMR (400 MHz, $CDCl_3$): $\delta$ = 8.93 (s, 1H), 8.63 (s, 2H), 4.23-4.17 (m, 4H), 1.80-1.72 (m, 4H), 1.47-1.24 (m, 20H), 0.89-0.86 (m, 6H), 0.54 (s, 9H).

[Example 2] (Synthesis of an aromatic compound of the present invention, represented by Formula (12) below)

Except that N,N'-dioctyl-2-[2-(trimethylsilyl)ethynyl]-1,4,5,8-naphthalenetetraca rboxylic acid diimide (4 mmol) was replaced with N,N'-bis(2-ethylhexyl)-2-[2-(trimethylsilyl)ethynyl]-1,4,5,8-naphthal enetetracarboxylic acid diimide (4 mmol), the process of Example 1 was followed to give an aromatic compound represented by Formula (12) below.

[Chemical Formula 15]

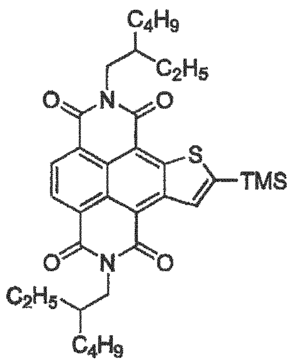

(12)

The yield of the aromatic compound represented by Formula (12) was 56%.

The nuclear magnetic resonance spectrum of the aromatic compound represented by Formula (12) is given below.
$^1$H-NMR (400 MHz, $CDCl_3$): $\delta$ = 8.95 (s, 1H), 8.67 (s, 2H), 4.22-4.10 (m, 4H), 2.04-1.92 (m, 2H), 1.43-1.24 (m, 16H), 0.96-0.86 (m, 12H), 0.54 (s, 9H).

[Example 3] (Synthesis of an aromatic compound of the present invention, represented by Formula (13) below)

In a nitrogen atmosphere, the aromatic compound represented by Formula (11) obtained in Example 1 (1.6 mmol), acetic acid (1.2 mL), and tetrahydrofuran (120 mL) were fed into a 300-mL eggplant-shaped recovery flask and cooled down to 0°C. Tetra-n-butylammonium fluoride (approximately 1.0 mol/L tetrahydrofuran solution, 16 mL) was added to the reaction liquid, which was then heated to room temperature and stirred for 3 hours. The reaction liquid was diluted with methanol (120 mL), and the solid deposit was filtered off. The filtered solid was rinsed with methanol and purified by silica gel column chromatography. Thus obtained was an orange, solid, aromatic compound represented by Formula (13) below.

[Chemical Formula 16]

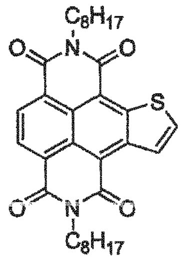

(13)

The yield of the aromatic compound represented by Formula (13) was 90%.

The nuclear magnetic resonance spectrum of the aromatic compound represented by Formula (13) is given below.

$^1$H-NMR (400 MHz, $CDCl_3$): $\delta$ = 8.96 (d, 1H), 8.84 (s, 2H), 8.16 (d, 1H), 4.29-4.24 (m, 4H), 1.82-1.75 (m, 4H), 1.48-1.29 (m, 20H), 0.89-0.86 (m, 6H).

[Example 4] (Synthesis of an aromatic compound of the present invention, represented by Formula (14) below)

In a nitrogen atmosphere, the aromatic compound represented by Formula (11) obtained in Example 1 (1.6 mmol), bromine (0.4 mL), and dichloromethane (40 mL) were fed into a 100-mL eggplant-shaped recovery flask, and stirred at 40°C for 15 hours. An aqueous solution of sodium hydrogen sulfite was added to this reaction liquid, and an organic layer was extracted with dichloromethane. The extracted organic layer was distillated to dryness, and the obtained solid was purified by silica gel column chromatography. Thus obtained was an orange, solid, aromatic compound represented by Formula (14) below.

[Chemical Formula 17]

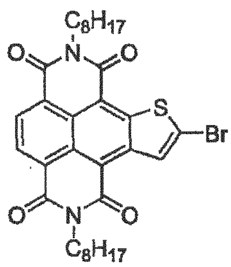

(14)

The yield of the aromatic compound represented by Formula (14) was 76%.

The nuclear magnetic resonance spectrum of the aromatic compound represented by Formula (14) is given below.

$^1$H-NMR (400 MHz, $CDCl_3$): $\delta$ = 8.64 (s, 2H), 8.58 (s, 1H), 4.15-4.10 (m, 4H), 1.77-1.69 (m, 4H), 1.46-1.28 (m, 20H), 0.89-0.86 (m, 6H).

[Example 5] (Synthesis of an aromatic compound of the present invention, represented by Formula (15) below)

Except that the aromatic compound represented by Formula (11) obtained in Example 1 was replaced with the one represented by Formula (12) obtained in Example 2, the process of Example 4 was followed to give an aromatic compound represented by Formula (15) below.

[Chemical Formula 18]

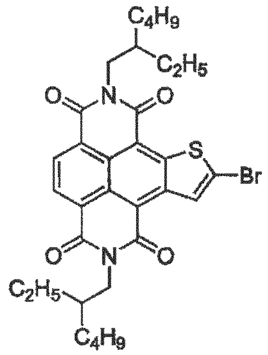

(15)

The yield of the aromatic compound represented by Formula (15) was 64%.

The nuclear magnetic resonance spectrum of the aromatic compound represented by Formula (15) is given below.

$^1$H-NMR (400 MHz, $CDCl_3$): δ = 8.88 (s, 2H), 8.78 (s, 1H), 4.22-4.11 (m, 4H), 2.02-1.90 (m, 2H), 1.43-1.31 (m, 16H), 0.97-0.87 (m, 12H).

[Example 6] (Synthesis of an aromatic compound of the present invention, represented by Formula (16) below)

In a nitrogen atmosphere, the aromatic compound represented by Formula (15) obtained in Example 5 (0.18 mmol), 1,3,5-tris(4,4,5,5-tetramethyl-1,3,2-dioxaborolane-2-yl)benzene (21 mg), tetrakis(triphenylphosphine)palladium (8 mg), an aqueous sodium carbonate solution (2 mol/L, 0.75 mL), and 1,4-dioxane (10 mL) were fed into a 50-mL eggplant-shaped recovery flask, stirred, and heated to reflux for 36 hours. The reaction liquid was cooled down to room temperature and poured into water (100 mL). The solid deposit was filtered off. The filtered solid was rinsed with water and methanol, and purified by silica gel column chromatography. Thus obtained was an orange, solid, aromatic compound represented by Formula (16) below.

[Chemical Formula 19]

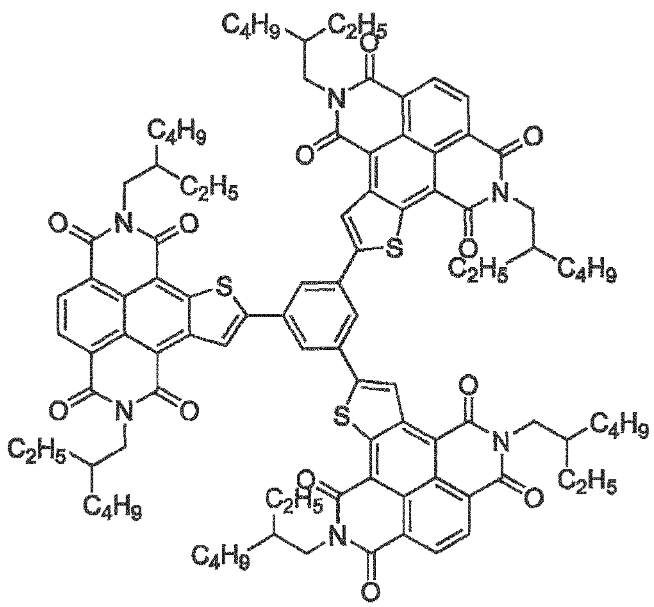

(16)

The yield of the aromatic compound represented by Formula (16) was 58%.

The nuclear magnetic resonance spectrum of the aromatic compound represented by Formula (16) is given

below.
$^1$H-NMR (400 MHz, $CDCl_3$): $\delta$ = 9.22 (s, 3H), 8.57 (d, 3H), 8.53 (d, 3H), 8.35 (s, 3H), 4.34-4.17 (m, 12H), 2.19-2.07 (m, 6H), 1.48-1.29 (m, 48H), 1.00-0.83 (m, 36H).

[Example 7] (Synthesis of an aromatic compound of the present invention, represented by Formula (17) below)

Except that the aromatic compound represented by Formula (15) obtained in Example 5 was replaced with the one represented by Formula (14) obtained in Example 4, and that 1,3,5-tris(4,4,5,5-tetramethyl-1,3,2-dioxaborolane-2-yl)benzene (21 mg) was replaced with N,N'-bis(3-decylpentadecyl)-2,6-bis(trimethylstannyl)naphtho[2,3-b-6,7-b']dithiophene-4,5,9,10-diimide (120 mg), the process of Example 6 was followed to give an aromatic compound represented by Formula (17) below.

[Chemical Formula 20]

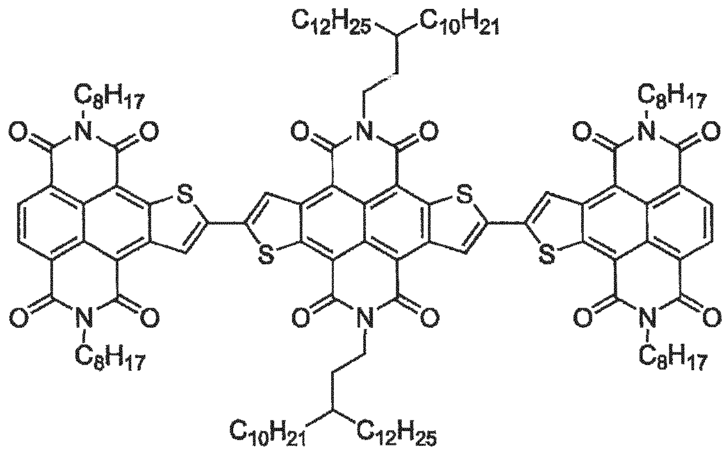

(17)

The yield of the aromatic compound represented by Formula (17) was 76%.
The nuclear magnetic resonance spectrum of the aromatic compound represented by Formula (17) is given below.
$^1$H-NMR (400 MHz, $CDCl_3$): $\delta$ = 9.40 (s, 2H), 9.30 (s, 2H), 8.41 (s,4H), 4.64 (t, J= 7.2 Hz, 4H), 4.37-4.32 (m, 8H), 2.13 (brs, 4H), 1.97 (m, 4H), 1.79-1.02 (m, 124H), 0.86 (t, J= 7.2 Hz, 12H), 0.78 (m, 12H).

[Example 8] (Synthesis of an aromatic compound of the present invention, represented by Formula (18) below)

Except that the aromatic compound represented by Formula (14) obtained in Example 4 was replaced with the one represented by Formula (15) obtained in Example 5, the process of Example 7 was followed to give an aromatic compound represented by Formula (18) below.

[Chemical Formula 21]

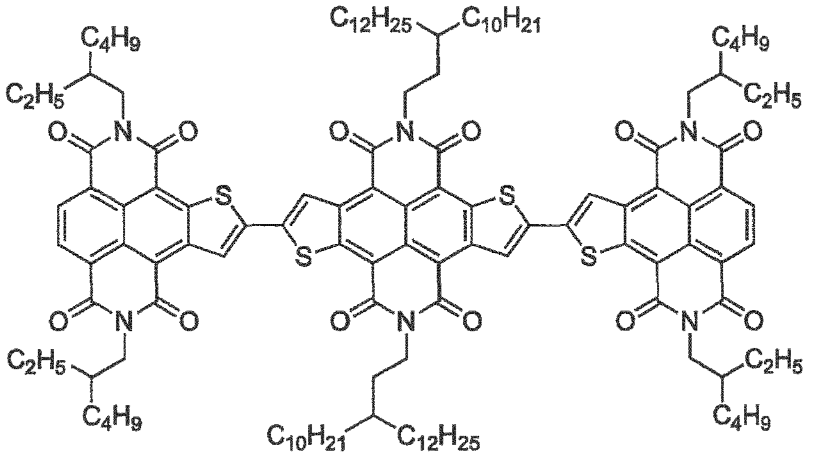

(18)

The yield of the aromatic compound represented by Formula (18) was 52%.

The nuclear magnetic resonance spectrum of the aromatic compound represented by Formula (18) is given below.

$^1$H-NMR (400 MHz, $CDCl_3$): δ = 9.39 (s, 2H), 9.33 (s, 2H), 8.45 (s, 4H), 4.60 (t, J= 7.2 Hz, 4H), 4.35-4.29 (m, 8H), 2.17 (brs, 4H), 2.09 (q, *J* = 7.2 Hz, 4H), 1.74-0.97 (m, 124H), 0.88 (t, J= 7.2 Hz, 12H), 0.77 (t, *J* = 7.2 Hz, 12H).

[Example 9] (Synthesis of an aromatic compound of the present invention, represented by Formula (19) below)

Except that 1,3,5-tris(4,4,5,5-tetramethyl-1,3,2-dioxaborolane-2-yl)benzene (21 mg) was replaced with 2,7-bis(4,4,5,5-tetramethyl-1,3,2-dioxaborolane-2-yl)-9,9-di-n-octylfl uorene (58 mg), the process of Example 6 was followed to give an aromatic compound represented by Formula (19) below.

[Chemical Formula 22]

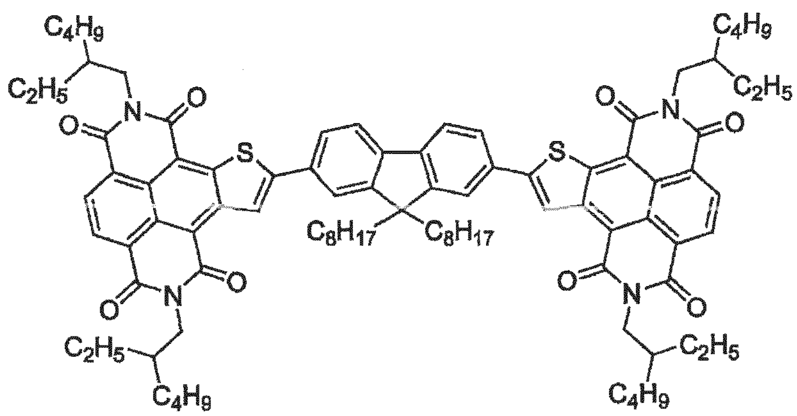

(19)

The yield of the aromatic compound represented by Formula (19) was 56%.

The nuclear magnetic resonance spectrum of the aromatic compound represented by Formula (19) is given below.

$^1$H-NMR (400 MHz, $CDCl_3$): δ = 9.29 (s, 2H), 8.82-8.77 (dd, 4H), 8.11 (d, 2H), 7.99 (s, 2H), 7.91 (d, 2H), 4.34-4.17 (m, 8H), 2.23 (m, 4H), 2.06 (d, 4H), 1.54-1.43 (m, 30H), 1.22-1.05 (m, 24H), 0.97 (t, 12H), 0.89 (t, 12H), 0.71 (m, 8H).

[Example 10] (Synthesis of an aromatic compound of the present invention, represented by Formula (20) below)

Except that 1,3,5-tris(4,4,5,5-tetramethyl-1,3,2-dioxaborolane-2-yl)benzene (21 mg) was replaced with 9-(9-heptadecanyl)-2,7-bis(4,4,5,5-tetramethyl-1,3,2-dioxaborolane-2 -yl)carbazole (59 mg), the process of Example 6 was followed to give an aromatic compound represented by Formula (20) below.

[Chemical Formula 23]

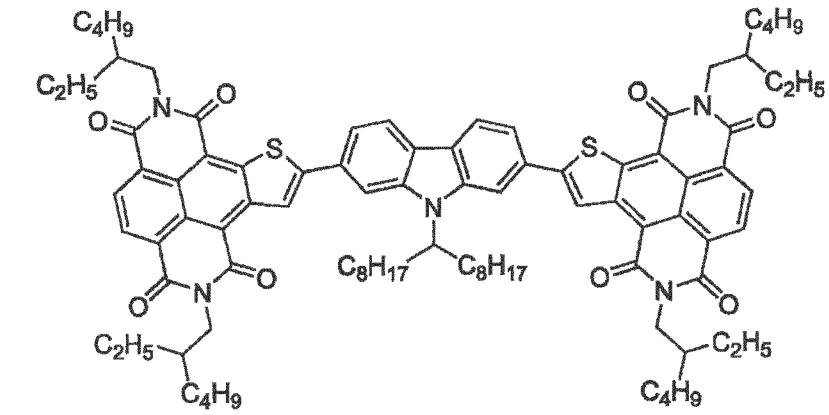

(20)

The yield of the aromatic compound represented by Formula (20) was 42%.

The nuclear magnetic resonance spectrum of the aromatic compound represented by Formula (20) is given below.

$^1$H-NMR (400 MHz, $CDCl_3$): $\delta$ = 9.28 (s, 2H), 8.81-8.76 (m, 4H), 8.21-7.94 (m, 6H), 4.81 (m, 1H), 4.26 (m, 8H), 2.52 (m, 2H), 2.31-2.01 (m, 6H), 1.53-1.34 (m, 32H), 1.22-1.05 (m, 24H), 0.97-0.89 (m, 28H), 0.89 (m, 6H).

[Example 11] (Synthesis of an aromatic compound of the present invention, represented by Formula (21) below)

Except that 1,3,5-tris(4,4,5,5-tetramethyl-1,3,2-dioxaborolane-2-yl)benzene (21 mg) was replaced with 2,2',7,7'-tetrakis(4,4,5,5-tetramethyl-1,3,2-dioxaborolane-2-yl)-9,9'-s pirobi[9H-fluorene] (36 mg), the process of Example 6 was followed to give an aromatic compound represented by Formula (21) below.

[Chemical Formula 24]

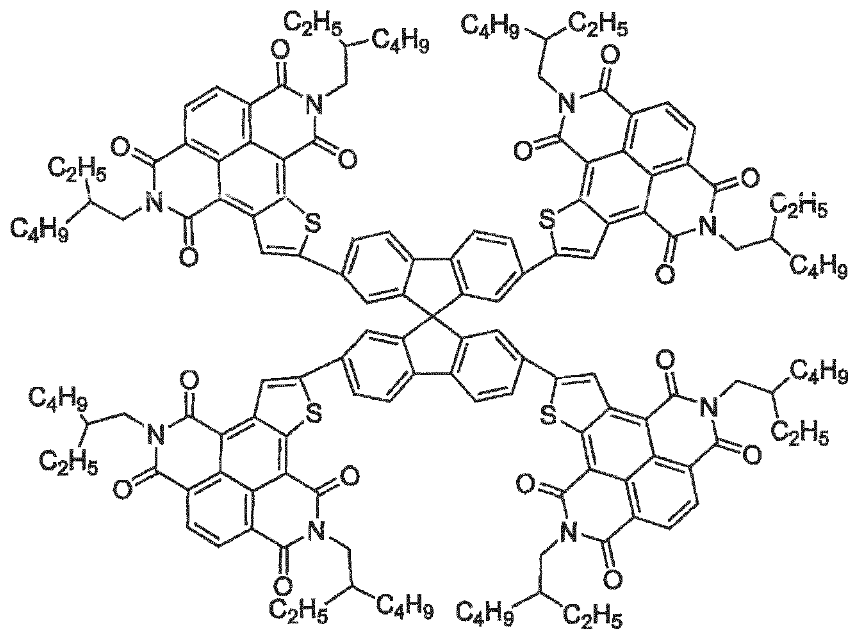

(21)

The yield of the aromatic compound represented by Formula (21) was 59%.

The nuclear magnetic resonance spectrum of the aromatic compound represented by Formula (21) is given

below.
$^1H$-NMR (400 MHz, $CDCl_3$): $\delta$ = 9.15-9.10 (m, 4H), 8.74-8.71 (m, 8H), 8.30-7.98 (m, 12H), 7.47-7.38 (m, 2H), 4.16 (m, 16H), 1.96 (m, 8H), 1.53-1.25 (m, 64H), 0.91-0.82 (m, 48H).

[Example 12] (Synthesis of an aromatic compound of the present invention, represented by Formula (22) below)

In a nitrogen atmosphere, the aromatic compound represented by Formula (14) obtained in Example 4 (0.15 mmol), bis(trimethyltin) (26 mg), tetrakis(triphenylphosphine)palladium (18 mg), and toluene (20 mL) were fed into a 50-mL eggplant-shaped recovery flask, heated to 105°C, and stirred for 10 hours. The reaction liquid was then cooled down to room temperature, and the solid deposit was filtered off. The filtered solid was recrystallized with use of o-dichlorobenzene to give a red, solid, aromatic compound represented by Formula (22) below.

[Chemical Formula 25]

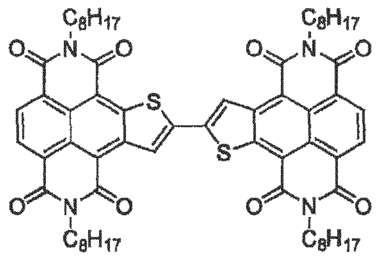

(22)

The yield of the aromatic compound represented by Formula (22) was 73%.
The nuclear magnetic resonance spectrum of the aromatic compound represented by Formula (22) is given below.
$^1H$-NMR (400 MHz, o-dichlorobenzene-d4): $\delta$ = 9.53 (s, 2H), 8.65 (s, 4H), 4.34-4.17 (m, 8H), 1.97-1.94 (m, 8H), 1.57-1.30 (m, 40H), 1.00-0.88 (m, 12H).

[Example 13] (Synthesis of an aromatic compound of the present invention, represented by Formula (23) below)

Except that the aromatic compound represented by Formula (14) obtained in Example 4 was replaced with the one represented by Formula (15) obtained in Example 5, the process of Example 12 was followed to give an aromatic compound represented by Formula (23).

[Chemical Formula 26]

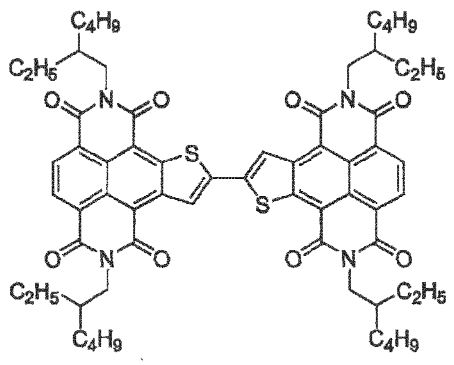

(23)

The yield of the aromatic compound represented by Formula (23) was 74%.
The nuclear magnetic resonance spectrum of the aromatic compound represented by Formula (23) is given below.
$^1H$-NMR (400 MHz, o-dichlorobenzene-d4): $\delta$ = 9.51 (s, 2H), 8.64 (s, 4H), 4.35-4.25 (m, 8H), 2.19-2.12 (m, 4H), 1.54-1.32 (m, 32H), 1.02-0.86 (m, 24H).

[Example 14] (Fabrication of an organic thin film transistor)

An organic thin film transistor having the top-contact bottom-gate structure shown in Fig. 1(b) was fabricated in this Example. To start with, a solution of the aromatic compound represented by Formula (17) obtained in Example 7 in 1,2,4-trichlorobenzene was applied by spin coating to an n-doped silicon wafer (a gate electrode 5 and a substrate 6) having a $SiO_2$ thermal oxide film (an insulator layer 4). An organic thin film (a semiconductor layer 2) was thereby formed on the $SiO_2$ thermal oxide film.

On this organic thin film, a source electrode 1 and a drain electrode 3 were formed by vaccum deposition of Au with use of a shadow mask. In the thus obtained organic thin film transistor, the channel had a length of 40 μm and a width of 1.5 mm.

[Example 15] (Fabrication of an organic thin film transistor)

Except that the aromatic compound represented by Formula (17) was replaced with the one represented by Formula (22) obtained in Example 12, the process of Example 14 was followed to give an organic thin film transistor.

[Example 16] (Fabrication of an organic thin film transistor)

Except that the aromatic compound represented by Formula (17) was replaced with the one represented by Formula (23) obtained in Example 13, the process of Example 14 was followed to give an organic thin film transistor.

[Evaluation of characteristics of organic thin film transistors]

Performance of an organic thin film transistor depends on the current value (the source-drain current value) flowing across the source electrode 1 and the drain electrode 3 when an electrical potential is applied across the source electrode 1 and the drain electrode 3 as well as to the gate electrode 5. Measurement of the current value can determine carrier mobility, which is one of the transistor characteristics. Carrier mobility can be calculated by Equation (a) below, which represents electrical characteristics of the type of carriers generated in the semiconductor layer 2 when a gate field is applied to the $SiO_2$ thermal oxide film serving as the insulator layer 4.

$$Id = Z \times \mu \times Ci(Vg - Vt)^2/2L \qquad (a)$$

wherein Id represents the saturated source-drain current value (A), Z represents the channel width (m), Ci represents the capacitance (F) of the insulator layer 4, Vg represents the gate potential (V), Vt represents the threshold potential (V), L represents the channel length (m), μ represents the carrier mobility to be determined ($cm^2/Vs$). Ci is determined by the dielectric constant of the employed $SiO_2$ thermal oxide film. Z and L are determined by the device structure of the organic thin film transistor. Id and Vg are determined when the current value of the organic thin film transistor is measured. Vt is obtainable from Id and Vg. The carrier mobility at various gate potentials can be obtained by substitution of the respective values into Equation (a). Characteristics of the organic thin film transistors were evaluated by Semiconductor Parameter Analyzer, Model 4200, manufactured by Keithley Instruments, Inc., wherein the drain voltage Vd = 40 V was applied across the source electrode 1 and the drain electrode 3.

Characteristics of the organic thin film transistors obtained in Examples 14-16 above were evaluated in the above-described manner. Fig. 3 shows transmission characteristics of the organic thin film transistor obtained in Example 16 using the aromatic compound represented by Formula (23). Table 1 shows evaluation results of the characteristics of the organic thin film transistors obtained in Examples 14-16.

[Table 1]

| Evaluation results of characteristics of organic thin film transistors | | | | |
|---|---|---|---|---|
| | Aromatic compound | Carrier mobility ($cm^2V^{-1}s^{-1}$) | On/off ratio | Threshold potential Vt (V) |
| Example 14 | Formula (17) | 0.12 | $10^4$ | -9.1 |
| Example 15 | Formula (22) | 0.23 | $10^7$ | 18.1 |
| Example 16 | Formula (23) | 0.43 | $10^8$ | -9.0 |

[Example 17] (Fabrication of organic thin film photovoltaic cell and evaluation of characteristics thereof)

As an embodiment of the organic photoelectric transducer, an organic thin film photovoltaic cell was fabricated, using the aromatic compound represented by Formula (19) obtained in Example 9. The organic thin film photovoltaic cell was based on the organic photoelectric transducer shown in Fig. 2, but with the insulation unit 11 removed. Specifically, the substrate was a glass substrate (a substrate 15) on which an ITO film (a bottom electrode 14) had been patterned as the negative electrode. This glass substrate was rinsed thoroughly, and then subjected to UV-ozone treatment. Next, a solution of 0.5M zinc acetate (II) dihydrate and ethanolamine in 2-methoxyethanol was applied to the ITO-film-side surface of the glass substrate, by spin coating at 3000 rpm for 30 seconds. The resulting glass substrate was heated at 200°C for 30 minutes to form a ZnO film (a part of a photoelectric conversion unit 13) which serves as an electron transport layer or an electron extraction layer.

The glass substrate provided with the ZnO film was set in a glovebox. In the glovebox, a chlorobenzene solution containing the aromatic compound represented by Formula (19) obtained in Example 9 and a compound represented by Formula (24) below at a mass ratio of 1:1 was applied to the surface of the ZnO film by spin coating to give a 100-nm-thick photoelectric conversion layer (a photoactive layer) (a different part of the photoelectric conversion unit 13). Thereafter, as a hole transport layer or a hole extraction layer, a $MoO_3$ film (a further different part of the photoelectric conversion unit 13) was formed on the surface of the photoelectric conversion layer. The thickness of the $MoO_3$ film was 7.5 nm. On the surface of this $MoO_3$ film, a Ag film was formed as a positive electrode (an upper electrode 12) by vacuum vapor deposition of Ag by resistive heating. The thickness of the Ag film was 100 nm. Fabricated through these steps was an organic photoelectric transducer (1) of the present invention. The organic photoelectric transducer (1) was made in a size of 4-mm cube.

[Chemical Formula 27]

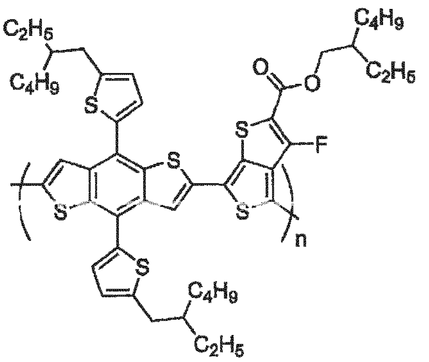

(24)

Using a solar simulator (Model: XES-40S1, manufactured by SAN-EI ELECTRIC CO.,LTD., AM1.5G filter, irradiance 100 mW/cm$^2$), the thus obtained organic photoelectric transducer (1) was irradiated with constant light. Generated electric current and voltage were measured. The measurement result is shown in Fig. 4. Based on the result shown in Fig. 4, the short-circuit current density Jsc (mA/cm$^2$), the open voltage Voc(V), and the form factor FF were obtained. The photoelectric conversion coefficient η was calculated with Jsc, Voc, and FF by Equation (b) below. The results are given in Table 2.

$$\eta = (Jsc \times Voc \times FF)/100 \qquad (b)$$

[Example 18] (Fabrication of an organic thin film photovoltaic cell and evaluation of characteristics thereof)

Except that the aromatic compound represented by Formula (19) obtained in Example 9 was replaced with the one represented by Formula (21) obtained in Example 11, and that the thickness of the photoelectric conversion layer was changed to 100 nm, the process of Example 17 was followed to fabricate an organic photoelectric transducer (2) of the present invention. Current density-voltage characteristics of the organic photoelectric transducer (2) were measured in the same manner as in Example 17. The measurement result is shown in Fig. 5. Based on the result shown in Fig. 5, Jsc, Voc, FF and η were obtained. The results are given in Table 2.

[Example 19] (Fabrication of an organic thin film photovoltaic cell and evaluation of characteristics thereof)

Except that the compound represented by Formula (24) was replaced with the one represented by Formula (25) below, and that the chlorobenzene solution contained the aromatic compound represented by Formula (21) and the compound represented by Formula (25) at a mass ratio of 4:5, the process of Example 18 was followed to fabricate an organic photoelectric transducer (3) of the present invention. Current density-voltage characteristics of the organic photoelectric transducer (3) were measured in the same manner as in Example 18. The measurement result is shown in Fig. 6. Based on the result shown in Fig. 6, Jsc, Voc, FF and η were obtained. The results are given in Table 2.

[Chemical Formula 28]

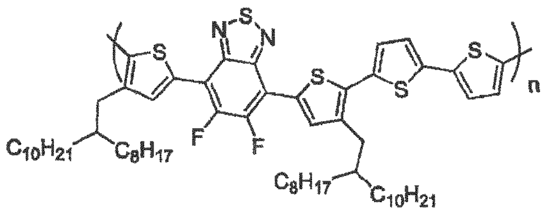

(25)

Table 2

| Evaluation results of organic thin film photovoltaic cells | | | | | |
|---|---|---|---|---|---|
| | Aromatic compound/ Compound | Jsc ($mA/cm^2$) | Voc (V) | FF | η (%) |
| Ex.17 | Formula(19)/Formula(24) | 5.74 | 0.83 | 0.47 | 2.26 |
| Ex.18 | Formula(21)/Formula(24) | 10.29 | 0.80 | 0.69 | 5.68 |
| Ex.19 | Formula(21)/Formula(25) | 11.58 | 0.87 | 0.70 | 7.05 |

The above results indicated that the organic semiconductor devices each using the organic thin film which contained the aromatic compound represented by General Formula (1) according to the present invention showed excellent characteristics in the organic thin film transistors and the organic photoelectric transducers. As clarified by these results, the present invention can fabricate organic semiconductor devices with higher performance and can thereby extend the range of applicable processes and applications. The present invention is thus industrially valuable.

[Industrial Applicability]

The organic thin film containing the aromatic compound according to the present invention is excellent in N-type transistor characteristics and photoelectric conversion characteristics. The aromatic compound according to the present invention is expected to be applied, as an organic thin film transistor, to a memory circuit device, a signal driver circuit device, a signal processing circuit device, and other digital devices, to be applied, as an organic photoelectric transducer, to an organic thin film photovoltaic cell, an organic imaging element, an optical sensor, a photon counter, and other various devices, and further to be applied to a solar battery, a camera, a video camera, an infrared camera, and other various devices using the organic thin film transistor or the organic photoelectric transducer.

[Reference Signs List]

| | |
|---|---|
| 1 | source electrode |
| 2 | semiconductor layer |
| 3 | drain electrode |
| 4 | insulator layer |
| 5 | gate electrode |
| 6 | substrate |
| 7 | protective layer |
| 10A-10F | organic thin film transistor |

| | |
|---|---|
| 11 | insulation unit |
| 12 | upper electrode |
| 13 | photoelectric conversion unit |
| 14 | bottom electrode |
| 15 | substrate |

The present invention can be embodied and practiced in other different forms without departing from the spirit and essential characteristics of the present invention. Therefore, the above-described embodiments are considered in all respects as illustrative and not restrictive. The scope of the invention is indicated by the appended claims rather than by the foregoing description. All variations and modifications falling within the equivalency range of the appended claims are intended to be embraced therein.

The present application claims priority to Japanese Patent Application No. 2016-137822, filed on July 12, 2016. The contents of all printed publications, patents, and patent applications (including the Japanese patent application mentioned just above) cited in the description are incorporated herein by reference in their entirety.

## Claims

1. An aromatic compound represented by General Formula (1),

[Chemical Formula 1]

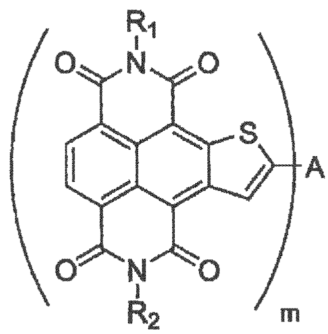

(1)

wherein each of $R_1$ and $R_2$ independently represents a hydrogen atom, a saturated hydrocarbon group having 1 to 30 carbon atoms, a saturated fluorohydrocarbon group having 1 to 30 carbon atoms, an aryl group, or a styryl group,

wherein the saturated hydrocarbon group having 1 to 30 carbon atoms, the saturated fluorohydrocarbon group having 1 to 30 carbon atoms, the aryl group, and the styryl group may have a substituent,

wherein m is an integer from 1 to 6,

wherein: when m is 1, A represents a hydrogen atom, a halogen atom, a hydrocarbon-trisubstituted silyl group, an aryl group, or a heteroaryl group; when m is 2, A represents a direct bond, or a divalent linking group having an aromatic ring or a heterocyclic ring; and when m is from 3 to 6, A represents a tri- to hexavalent linking group having an aromatic ring or a heterocyclic ring,

wherein, if the aromatic compound contains more than one Ri, $R_1$ may be identical to or different from each other; and if the aromatic compound contains more than one $R_2$, $R_2$ may be identical to or different from each other.

2. The aromatic compound according to claim 1, wherein m is 2, and A represents a direct bond.

3. The aromatic compound according to claim 1, wherein m is 2, and A represents a divalent linking group having an aromatic ring or a heterocyclic ring.

4. The aromatic compound according to claim 1, wherein m is 3, and A represents a trivalent linking group having an aromatic ring or a heterocyclic ring.

5. The aromatic compound according to claim 1, wherein m is 4, and A represents a tetravalent linking group having an aromatic ring or a heterocyclic ring.

**6.** The aromatic compound according to claim 1, wherein m is 6, and A represents a hexavalent linking group having an aromatic ring or a heterocyclic ring.

**7.** The aromatic compound according to any one of claims 1 to 6, wherein $R_1$ and $R_2$ represent an identical alkyl group having 1 to 30 carbon atoms.

**8.** The aromatic compound according to any one of claims 1 to 6, wherein $R_1$ and $R_2$ represent an identical aryl group having 6 to 12 carbon atoms.

**9.** An organic semiconductor material comprising the aromatic compound according to any one of claims 1 to 8.

**10.** A thin film forming composition comprising the aromatic compound according to any one of claims 1 to 8, and an organic solvent.

**11.** An organic thin film comprising the aromatic compound according to any one of claims 1 to 8.

**12.** An organic semiconductor device comprising the organic thin film according to claim 11.

**13.** The organic semiconductor device according to claim 12, wherein the organic semiconductor device is an organic photoelectric transducer.

**14.** The organic semiconductor device according to claim 12, wherein the organic semiconductor device is an organic thin film transistor.

Fig.1 (a)

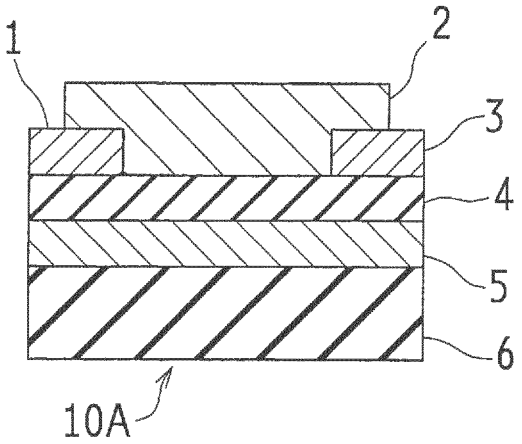


Fig.1 (b)

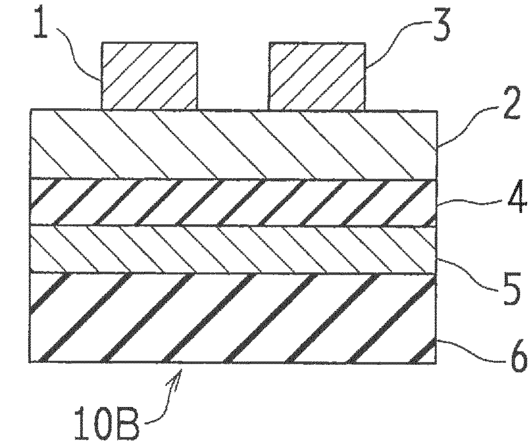


Fig.1 (c)

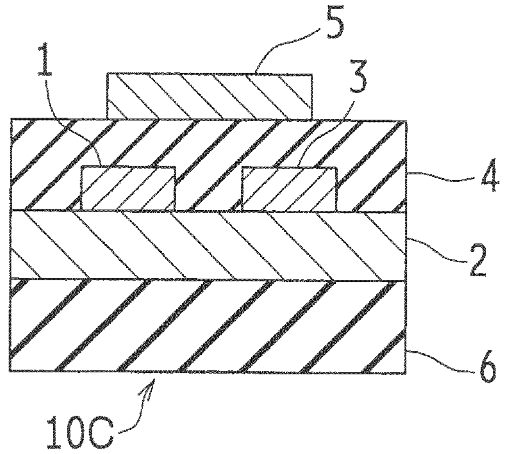


Fig.1 (d)

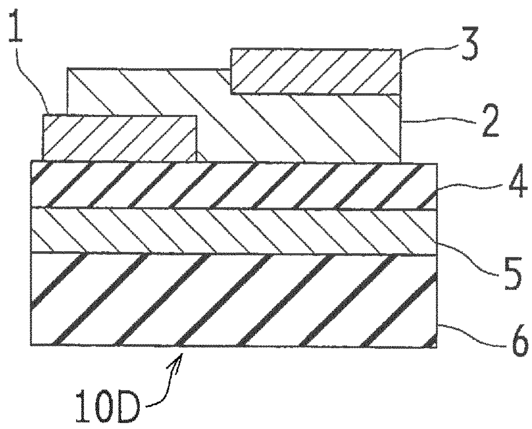


Fig.1 (e)

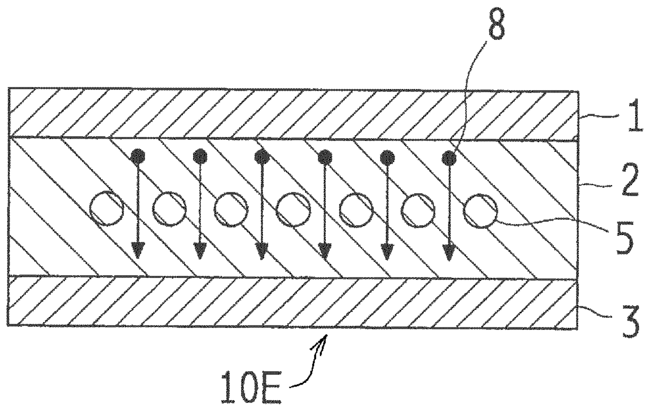


Fig.1 (f)

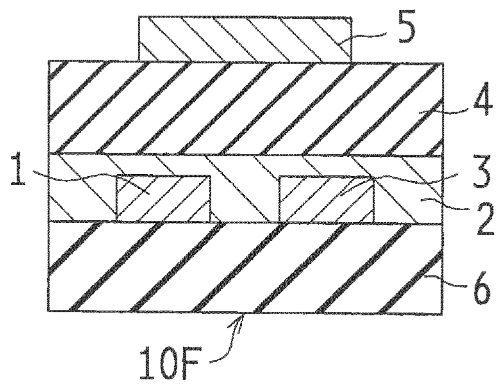

Fig.2

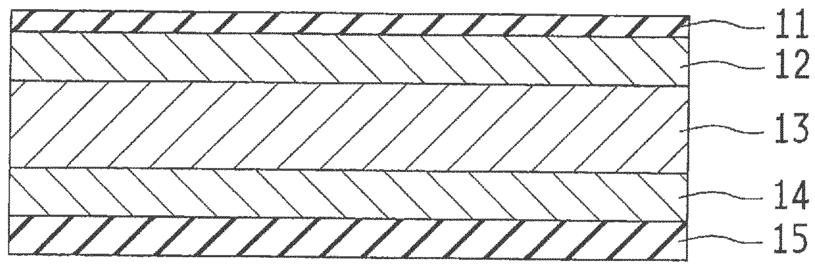
11
12
13
14
15

Fig.3

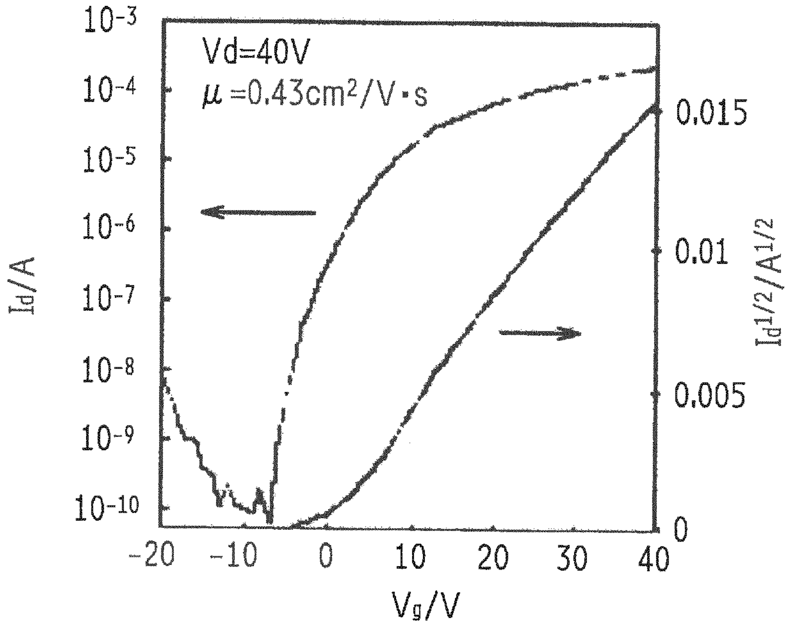
Vd=40V
μ=0.43cm2/V·s
Id/A
Id1/2/A1/2
Vg/V
10-3
10-4
10-5
10-6
10-7
10-8
10-9
10-10
0.015
0.01
0.005
0
-20
-10
0
10
20
30
40

Fig.4

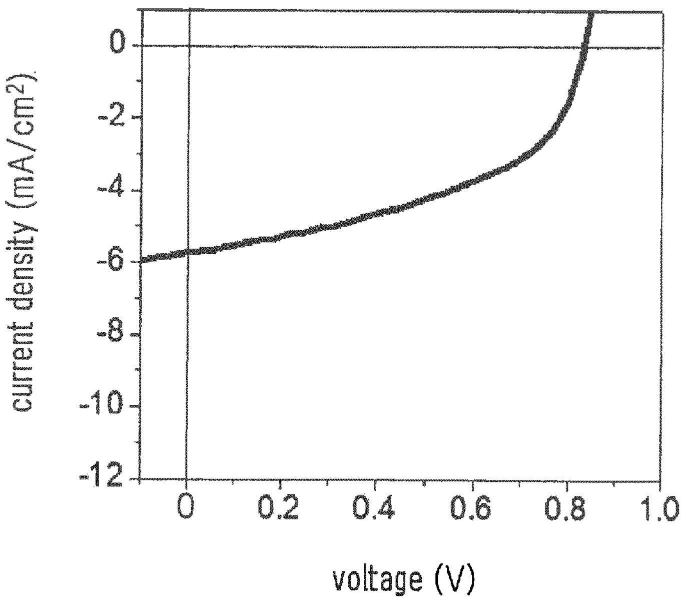
0
-2
-4
-6
-8
-10
-12
current density (mA/cm2)
0
0.2
0.4
0.6
0.8
1.0
voltage (V)

Fig.5

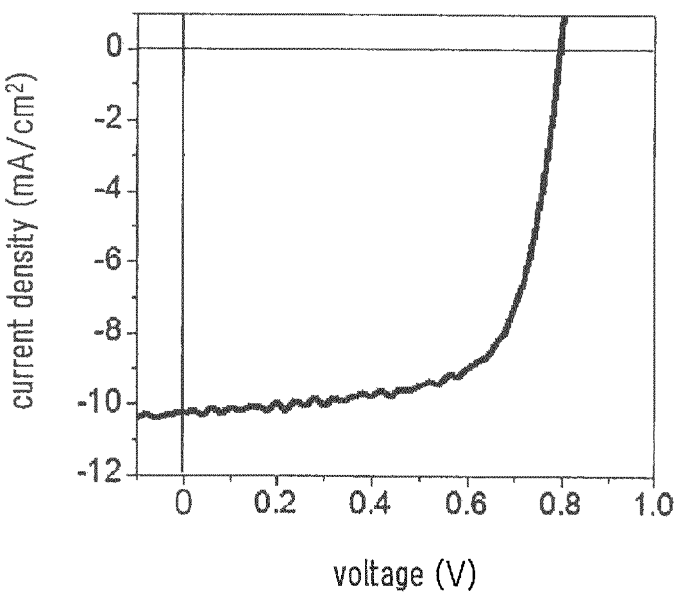
0
-2
-4
-6
-8
-10
-12
current density (mA/cm2)
0
0.2
0.4
0.6
0.8
1.0
voltage (V)

Fig.6

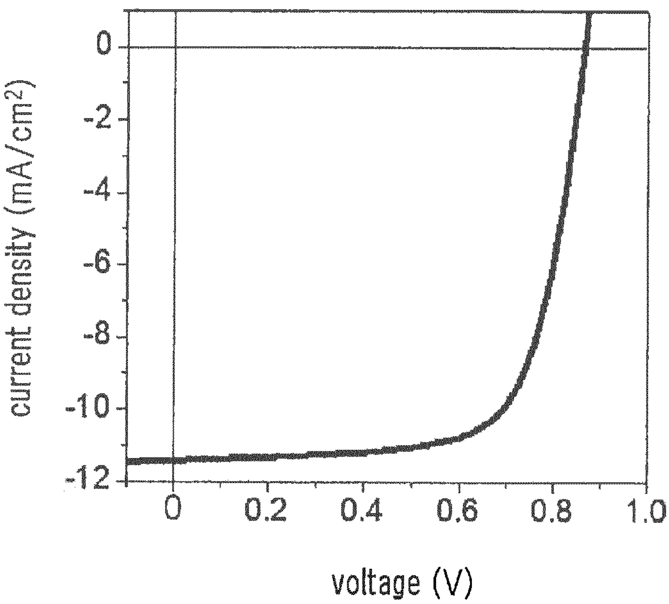

**INTERNATIONAL SEARCH REPORT**

International application No.
PCT/JP2017/025308

A. CLASSIFICATION OF SUBJECT MATTER
*C07D495/16*(2006.01)i, *C07D519/00*(2006.01)i, *C07F7/10*(2006.01)i, *H01L29/786*(2006.01)i, *H01L51/05*(2006.01)i, *H01L51/30*(2006.01)i, *H01L51/46*(2006.01)i

According to International Patent Classification (IPC) or to both national classification and IPC

B. FIELDS SEARCHED

Minimum documentation searched (classification system followed by classification symbols)
C07D495/16, C07D519/00, C07F7/10, H01L29/786, H01L51/05, H01L51/30, H01L51/46

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY/MARPAT(STN)

C. DOCUMENTS CONSIDERED TO BE RELEVANT

| Category* | Citation of document, with indication, where appropriate, of the relevant passages | Relevant to claim No. |
|---|---|---|
| A | JP 2013-508311 A (Institute of Organic Chemistry Academia Sinicia, Shanghai, China), 07 March 2013 (07.03.2013), claims & US 2012/0253045 A1 & WO 2011/047624 A1 & EP 2492271 A1 & CN 101693719 A & KR 10-2012-0085825 A | 1-14 |
| A | WO 2014/178415 A1 (Riken, Japan), 06 November 2014 (06.11.2014), claims (Family: none) | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 August 2017 (15.08.17) | 22 August 2017 (22.08.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2017/025308

C (Continuation). DOCUMENTS CONSIDERED TO BE RELEVANT

| Category* | Citation of document, with indication, where appropriate, of the relevant passages | Relevant to claim No. |
|---|---|---|
| A | JP 2015-87501 A (Kyocera Document Solutions Inc.), 07 May 2015 (07.05.2015), claims & US 2015/0118607 A1 & EP 2869125 A1 & CN 104597729 A | 1-14 |
| P,X | Chen, W. et al., Selective thionation of naphtho[2,3-b]thiophene diimide: tuning of the optoelectronic properties and packing structure, Org. Chem. Front., 2017.01.17, vol.4, p.704-710 | 1-14 |
| P,X | Chen, W. et al., Naphtho[2,3-b]thiophene diimide (NTI): a mono-functionalisable core-extended naphthalene diimide for electron-deficient architectures, J. Mater. Chem. C, 2016.09.01, vol.4, p.8879-8883 | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101885732 A
- WO 2014178415 A1
- JP 5887323 B
- JP 2016137822 A


### Non-patent literature cited in the description


- *Chemical Communications,* 2011, vol. 47, 10112-10114
- *Chemical Communications,* 2011, vol. 47, 11504-11506
- *Chemistry of Materials,* 2011, vol. 23 (5), 1204-1215
- *Journal of Materials Chemistry C,* 2013, vol. 1, 1087-1092
- *Organic & Biomolecular Chemistry,* 2012, vol. 10, 6455-6468